# FASCICULE DE BREVET EUROPEEN

(11) **EP 3 161 451 B1**
(45) Date de publication et mention de la délivrance du brevet: **07.06.2023**
(21) Numéro de dépôt: 15756182.0
(22) Date de dépôt: 23.06.2015
(51) Int. Cl.: G01N 15/14, G01N 15/10

(54) **SYSTÈME ET ENSEMBLE DE CYTOMÉTRIE EN FLUX, DISPOSITIF D'ANALYSE COMPRENANT UN TEL ENSEMBLE DE CYTOMÉTRIE ET ENSEMBLE COMPRENANT UN TEL SYSTÈME DE CYTOMÉTRIE**
DURCHFLUSSZYTOMETRIEANORDNUNG UND SYSTEM, ANALYSEVORRICHTUNG MIT EINER SOLCHEN ZYTOMETRIEANORDNUNG UND ANORDNUNG MIT SOLCH EINEM ZYTOMETRIESYSTEM
FLOW CYTOMETRY ASSEMBLY AND SYSTEM, ANALYSING DEVICE COMPRISING SUCH A CYTOMETRY ASSEMBLY AND ASSEMBLY COMPRISING SUCH A CYTOMETRY SYSTEM

(30) Priorité: 30.06.2014 FR 1456230
(43) Date de publication de la demande: 03.05.2017
(73) Titulaire: ARTEION, 75007 Paris (FR)
(72) Inventeur: ROUSSEAU, Alain, 75001 Paris (FR)
(74) Mandataire: Germain Maureau
(86) Numéro de dépôt international: PCT/FR2015/051677
(87) Numéro de publication internationale: WO 2016/001522

(56) Documents cités:
- EP-A1- 0 068 404
- FR-A1- 2 653 885
- US-A- 3 710 933
- US-A1- 2010 020 308
- US-A1- 2011 089 340

## Description

La présente invention concerne un système de cytométrie en flux destiné à l'analyse de particules biologiques.

Des systèmes de cytométrie selon l'art antérieur sont connus des documents EP 0 068 404 A1 et FR 2 653 885.

Le document FR 2 653 885 divulgue un système de cytométrie en flux comprenant :
- une cellule de mesure délimitant au moins en partie une chambre de mesure,
- un dispositif d'injection agencé pour injecter un flux de particules biologiques à analyser dans la chambre de mesure, le dispositif d'injection comprenant :
   - une buse d'injection délimitant une chambre interne et comprenant un orifice d'injection relié fluidiquement à la chambre de mesure,
   - un premier conduit d'alimentation débouchant dans la chambre interne et destiné à alimenter la chambre interne avec un échantillon liquide contenant les particules biologiques à analyser en suspension, et
   - un deuxième conduit d'alimentation débouchant dans la chambre interne et destiné à alimenter la chambre interne avec un premier fluide de gainage, la buse d'injection et le deuxième conduit d'alimentation étant configurés de telle sorte que le premier fluide de gainage introduit dans la chambre interne est apte à gainer hydrodynamiquement l'échantillon liquide introduit dans la chambre interne,
- un dispositif d'évacuation agencé pour évacuer à l'extérieur du système de cytométrie le flux de particules biologiques injecté dans la chambre de mesure, et
- un ensemble de mesure agencé pour mesurer au moins une propriété optique des particules biologiques à analyser, l'ensemble de mesure comportant :
   - un dispositif d'émission agencé pour émettre un faisceau lumineux en direction de la chambre de mesure et apte à croiser le flux de particules biologiques, le dispositif d'émission comprenant une source lumineuse agencée pour générer le faisceau lumineux, et
   - un dispositif de collecte agencé pour collecter des rayons lumineux issus de la chambre de mesure, et plus particulièrement des rayons
   lumineux diffusés ou diffractés par chaque particule biologique introduite dans la chambre de mesure et croisant le faisceau lumineux.

Le gainage hydrodynamique de l'échantillon liquide contenant les particules biologiques à analyser permet d'étirer l'échantillon liquide avant son passage à travers l'orifice d'injection, et donc d'une part de confiner précisément les particules biologiques et d'autre part d'optimiser le centrage du flux de particules biologiques dans la chambre de mesure. Ces dispositions permettent ainsi de faciliter le positionnement relatif du faisceau lumineux incident et du flux de particules biologiques, et de ce fait d'améliorer la qualité des mesures des propriétés optiques des particules biologiques à analyser.

Le système de cytométrie en flux décrit dans le document FR 2 653 885 permet en outre de limiter la consommation de liquides réactionnels du fait du faible volume de la chambre de mesure délimitée par la cellule de mesure.

Néanmoins, un tel système de cytométrie en flux requiert l'utilisation de systèmes de réglage onéreux et complexes pour aligner le faisceau lumineux incident sur le flux de particules biologiques.

De plus, les systèmes de réglage utilisés pour un tel système de cytométrie en flux présentent une précision insuffisante. Ainsi, les mesures optiques réalisées avec un tel système de cytométrie en flux peuvent encore être améliorée.

La présente invention vise à remédier à tout ou partie de ces inconvénients.

Le problème technique à la base de l'invention consiste notamment à fournir un système de cytométrie en flux qui soit de structure simple et économique, tout en permettant de réaliser des mesures optiques fiables.

A cet effet, la présente invention concerne un système de cytométrie en flux destiné à l'analyse de particules biologiques selon la revendication 1, le système comprenant :
- une cellule de mesure délimitant au moins en partie une chambre de mesure,
- un dispositif d'injection agencé pour injecter un flux de particules biologiques à analyser dans la chambre de mesure, le dispositif d'injection comprenant :
   - une buse d'injection délimitant une chambre interne et comprenant un orifice d'injection relié fluidiquement à la chambre de mesure,
   - un premier conduit d'alimentation débouchant dans la chambre interne et destiné à alimenter la chambre interne avec un échantillon liquide contenant les particules biologiques à analyser en suspension, et
   - un deuxième conduit d'alimentation débouchant dans la chambre interne et destiné à alimenter la chambre interne avec un premier fluide de gainage, la buse d'injection et le deuxième conduit d'alimentation étant configurés de telle sorte que le premier fluide de gainage introduit dans la chambre interne est apte à gainer hydrodynamiquement l'échantillon liquide introduit dans la chambre interne,
- un dispositif d'évacuation agencé pour évacuer à l'extérieur du système de cytométrie le flux de particules biologiques injecté dans la chambre de mesure,
- un troisième conduit d'alimentation relié fluidiquement à la chambre de mesure et destiné à alimenter la chambre de mesure avec un deuxième fluide de gainage, la chambre de mesure et le troisième conduit d'alimentation étant configurés de telle sorte que le deuxième fluide de gainage introduit dans la chambre de mesure est apte à gainer hydrodynamiquement le flux de particules biologiques dans la chambre de mesure,
- un ensemble de mesure agencé pour mesurer au moins une propriété optique des particules biologiques à analyser, telle que l'intensité de l'absorption des particules biologiques, l'ensemble de mesure comportant :
   - au moins un dispositif d'émission agencé pour émettre un faisceau lumineux en direction de la chambre de mesure et apte à croiser le flux de particules biologiques, l'au moins un dispositif d'émission comprenant une source lumineuse agencée pour générer le faisceau lumineux,
   - au moins un dispositif de collecte agencé pour collecter des rayons lumineux issus de la chambre de mesure, et
- un support sur lequel sont montés le dispositif d'injection, le dispositif d'évacuation, l'au moins un dispositif d'émission et l'au moins un dispositif de collecte, le support délimitant un logement de réception dans lequel est logée la cellule de mesure.

Le fait de monter les dispositifs d'émission et de collecte sur un même support, dit de référence, permet d'améliorer la stabilité et le positionnement relatif des dispositifs d'émission et de collecte, et donc la fiabilité des mesures optiques réalisées.

En outre, le fait de monter les dispositifs d'injection et d'évacuation sur un support de référence rend possible la réalisation des dispositifs d'injection et d'évacuation à partir de pièces moulées ou surmoulées de faibles précisions, ce qui permet de réduire les coûts de fabrication du système de cytométrie en flux selon la présente invention.

Il doit être noté que le gainage du flux de particules biologiques dans la chambre de mesure permet de maintenir centré et stabilisé le flux de particules biologique durant son trajet dans la chambre de mesure.

Selon un mode de réalisation de l'invention, l'au moins un dispositif d'émission est agencé pour émettre un faisceau laser.

Selon un mode de réalisation de l'invention, l'au moins un dispositif d'émission comporte un dispositif de focalisation agencé pour focaliser le faisceau lumineux dans la chambre de mesure et sur le flux de particules biologiques.

Selon un mode de réalisation de l'invention, le dispositif de focalisation comprend :
- une première partie de montage équipée d'un élément optique de focalisation disposé sur le chemin optique du faisceau lumineux,
- une deuxième partie de montage sur laquelle est montée la source lumineuse, les première et deuxième parties de montage du dispositif de focalisation étant déplaçables l'une par rapport à l'autre selon une première direction de déplacement sensiblement parallèle au chemin optique du faisceau lumineux, et
- un premier élément de réglage, tel qu'une vis micrométrique, agencé pour régler la position relative des première et deuxième parties de montage du dispositif de focalisation le long de la première direction de déplacement.

Selon un mode de réalisation de l'invention, l'élément optique de focalisation comporte une lentille de focalisation.

Selon un mode de réalisation de l'invention, le dispositif de focalisation comprend au moins un élément d'immobilisation agencé pour immobilier la première partie de montage par rapport au support, la deuxième partie de montage du dispositif de focalisation étant monté mobile par rapport à la première partie de montage du dispositif de focalisation.

Selon un mode de réalisation de l'invention, l'au moins un élément d'immobilisation comporte au moins une vis d'immobilisation.

Selon un mode de réalisation de l'invention, la première partie de montage du dispositif de focalisation délimite un conduit de guidage dans lequel est montée coulissante au moins une portion de la deuxième partie de montage du dispositif de focalisation.

Selon un mode de réalisation de l'invention, le premier élément de réglage comprend une première portion filetée agencée pour coopérer avec un premier alésage taraudé ménagé sur la première partie de montage du dispositif de focalisation, et une deuxième portion filetée agencée pour coopérer avec un deuxième alésage taraudé ménagé sur la deuxième partie de montage du dispositif de focalisation, les première et deuxième portions filetées présentant des filetages de pas différents.

Selon un mode de réalisation de l'invention, le système de cytométrie en flux comporte au moins un dispositif de réglage d'orientation, également nommé dispositif de réglage d'assiette, agencé pour régler l'orientation du faisceau lumineux émis par l'au moins un dispositif d'émission.

Selon un mode de réalisation de l'invention, le dispositif de réglage d'orientation est agencé pour régler l'orientation du faisceau lumineux émis par l'au moins un dispositif d'émission de telle sorte que le chemin optique du faisceau lumineux s'étende sensiblement orthogonalement à la direction d'écoulement du flux de particules biologiques.

Selon un mode de réalisation de l'invention, le dispositif de réglage d'orientation comporte :
- un coussin de réglage disposé entre le support et l'au moins un dispositif d'émission, le coussin de réglage étant au moins en partie élastiquement déformable, et
- un ensemble de déformation agencé pour déformer le coussin de compression de manière à régler l'orientation du faisceau lumineux émis par l'au moins un dispositif d'émission.

Selon un mode de réalisation de l'invention, le coussin de réglage est annulaire. Par exemple, le coussin de réglage délimite un passage central à travers lequel s'étend au moins une portion du dispositif d'émission.

Selon un mode de réalisation de l'invention, la première partie de montage du dispositif de focalisation comporte une portion d'appui agencée pour prendre appui contre le coussin de réglage.

Selon un mode de réalisation de l'invention, la portion d'appui de la première partie de montage comprend un orifice de passage à travers lequel s'étend l'au moins un élément d'immobilisation.

Selon un mode de réalisation de l'invention, l'ensemble de déformation est formé par l'au moins un élément d'immobilisation et la portion d'appui de la première partie de montage.

Selon un mode de réalisation de l'invention, le coussin de réglage comprend un orifice de passage à travers lequel s'étend l'au moins un élément d'immobilisation.

Selon un mode de réalisation de l'invention, l'au moins un dispositif de collecte comporte :
- une première partie de montage comprenant un premier élément optique de collecte,
- une deuxième partie de montage comprenant au moins un deuxième élément optique de collecte, les première et deuxième parties de montage du dispositif de collecte étant déplaçables l'une par rapport à l'autre selon une deuxième direction de déplacement, et
- un deuxième élément de réglage, tel qu'une vis micrométrique, agencé pour régler la position relative des première et deuxième parties de montage du dispositif de collecte le long de la deuxième direction de déplacement.

Selon un mode de réalisation de l'invention, l'au moins un dispositif de collecte comprend au moins un élément d'immobilisation agencé pour immobilier la première partie de montage du dispositif de collecte par rapport au support, la deuxième partie de montage du dispositif de collecte étant monté mobile par rapport à la première partie de montage du dispositif de collecte.

Selon un mode de réalisation de l'invention, la première partie de montage du dispositif de collecte délimite un conduit de guidage dans lequel est montée coulissante au moins une portion de la deuxième partie de montage du dispositif de collecte.

Selon un mode de réalisation de l'invention, le deuxième élément de réglage comprend une première portion filetée agencée pour coopérer avec un premier alésage taraudé ménagé sur la première partie de montage du dispositif de collecte, et une deuxième portion filetée agencée pour coopérer avec un deuxième alésage taraudé ménagé sur la deuxième partie de montage du dispositif de collecte, les première et deuxième portions filetées présentant des filetages de pas différents.

Selon un mode de réalisation de l'invention, le premier élément optique de collecte comporte une lentille optique qui peut par exemple former un collimateur.

Selon un mode de réalisation de l'invention, l'au moins un deuxième élément optique de collecte comporte au moins une fibre optique de collecte.

Selon un mode de réalisation de l'invention, la deuxième partie de montage du dispositif de collecte comprend une pluralité de fibres optiques de collecte. Selon un mode de réalisation de l'invention, la deuxième partie de montage du dispositif de collecte comprend une fibre optique de collecte centrale, et des fibres optiques de collecte périphériques. Par exemple, la fibre optique de collecte centrale est destinée à collecter les rayons lumineux issus de la chambre de mesure selon le chemin optique du faisceau lumineux incident, c'est-à-dire à 0°, et les fibres optiques de collecte périphériques sont destinées à collecter des rayons lumineux issus de la chambre de mesure sous de faibles angles, par exemple inférieurs à 15°. La deuxième partie de montage du dispositif de collecte peut par exemple comprendre au moins une fibre optique de collecte périphérique destinée à collecter des rayons lumineux issus de la chambre de mesure à un angle de l'ordre de 4° et au moins une fibre optique de collecte périphérique destinée à collecter des rayons lumineux issus de la chambre de mesure à un angle de l'ordre de 9°.

Selon un mode de réalisation de l'invention, le système de cytométrie en flux comprend un dispositif de mesure de variation d'impédance électrique agencé pour mesurer la variation d'impédance électrique générée par le passage des particules biologiques à travers l'orifice d'injection, le dispositif de mesure de variation d'impédance électrique comprenant une première et une deuxième électrodes disposées respectivement de part et d'autre de l'orifice d'injection, les première et deuxième électrodes étant destinées à être en contact électrique avec le flux de particules biologiques de manière à établir un champ électrique au travers de l'orifice d'injection. Un tel dispositif de mesure de variation d'impédance électrique permet de compter le nombre de particules biologiques passant par l'orifice d'injection, et également de déterminer la taille, et plus précisément le volume des particules biologiques.

Selon un mode de réalisation de l'invention, l'ensemble de mesure comprend une pluralité de dispositifs de collecte décalés angulairement par rapport à la cellule de mesure, et plus précisément par rapport à l'axe du flux de particules biologiques.

Selon un mode de réalisation de l'invention, l'au moins un dispositif de collecte est disposé sensiblement à l'opposé du dispositif d'émission par rapport à la cellule de mesure.

Selon un mode de réalisation de l'invention, le système de cytométrie en flux comprend une pluralité de dispositifs d'émission décalés angulairement par rapport à la cellule de mesure, et plus précisément par rapport à l'axe du flux de particules biologiques.

Selon un mode de réalisation de l'invention, le dispositif d'évacuation comporte un conduit d'évacuation relié fluidiquement à la chambre de mesure et destiné à évacuer le flux de particules biologiques injecté dans la chambre de mesure, et en outre le troisième conduit d'alimentation. Ces dispositions permettent de répartir de manière sensiblement symétrique les entrés et les sorties de fluides par rapport au support, et donc de faciliter l'assemblage du système de cytométrie en flux selon l'invention et de rendre plus aisé l'accès aux différentes entrées et sorties de fluide. Ces dispositions permettent également de faciliter la fabrication des dispositifs d'injection et d'évacuation, puisque certaines pièces constitutives de ces derniers peuvent alors être fabriquées à partir d'un même moule ou d'un moule pourvu d'inserts ou de pièces permettant d'adapter sa forme.

Selon un mode de réalisation de l'invention, le système de cytométrie à flux est conformé de telle sorte que la pression du deuxième fluide de gainage injecté dans la chambre de mesure est inférieure à la pression du premier fluide de gainage injecté dans la chambre interne.

Selon un mode de réalisation de l'invention, le dispositif d'injection comporte un premier conduit de refoulement relié fluidiquement à la chambre interne et destiné à refouler à l'extérieur du système de cytométrie le contenu de la chambre interne. Le premier conduit de refoulement est plus particulièrement destiné à refouler à l'extérieur du système de cytométrie un premier fluide de rinçage introduit dans la chambre interne via le deuxième conduit d'alimentation.

Selon un mode de réalisation de l'invention, le système de cytométrie en flux comprend en outre une première vanne de refoulement reliée fluidiquement au premier conduit de refoulement et mobile entre une position de fermeture dans laquelle la première vanne de refoulement empêche un écoulement de fluide de la chambre interne vers l'extérieur du système de cytométrie via le premier conduit de refoulement, et une position d'ouverture dans laquelle la première vanne de refoulement autorise un écoulement de fluide de la chambre interne vers l'extérieur du système de cytométrie via le premier conduit de refoulement.

Selon un mode de réalisation de l'invention, l'au moins un dispositif d'évacuation comporte un deuxième conduit de refoulement relié fluidiquement à la chambre de mesure et destiné à refouler à l'extérieur du système de cytométrie le contenu de la chambre de mesure. Le deuxième conduit de refoulement est plus particulièrement destiné à refouler à l'extérieur du système de cytométrie un deuxième fluide de rinçage introduit dans la chambre de mesure via le troisième conduit d'alimentation et le fluide à mesurer qui passe par l'orifice d'injection et provient de la chambre interne.

Selon un mode de réalisation de l'invention, le système de cytométrie en flux comprend en outre une deuxième vanne de refoulement reliée fluidiquement au deuxième conduit de refoulement et mobile entre une position de fermeture dans laquelle la deuxième vanne de refoulement empêche un écoulement de fluide de la chambre de mesure vers l'extérieur du système de cytométrie via le deuxième conduit de refoulement, et une position d'ouverture dans laquelle la deuxième vanne de refoulement autorise un écoulement de fluide de la chambre de mesure vers l'extérieur du système de cytométrie via le deuxième conduit de refoulement.

Selon un mode de réalisation de l'invention, la première vanne de refoulement et/ou la deuxième vanne de refoulement est une électrovanne.

Selon un mode de réalisation de l'invention, le support comporte au moins une première ouverture de passage à travers laquelle s'étend au moins une partie du dispositif d'émission, une deuxième ouverture de passage à travers laquelle s'étend au moins une partie de l'au moins un dispositif de collecte, une troisième ouverture de passage à travers laquelle s'étend au moins une partie du dispositif d'injection, et au moins une quatrième ouverture de passage à travers laquelle s'étend au moins une partie du dispositif d'évacuation, les première, deuxième, troisième et quatrième ouvertures de passage débouchant dans le logement de réception.

Selon un mode de réalisation de l'invention, les premier et deuxième conduits d'alimentation comportent respectivement une première et une deuxième extrémités débouchant dans la chambre interne, la deuxième extrémité étant plus éloignée de l'orifice d'injection que la première extrémité.

Selon un mode de réalisation de l'invention, le premier conduit d'alimentation comporte une première portion d'alimentation tubulaire débouchant dans la chambre interne, et le deuxième conduit d'alimentation comporte une deuxième portion d'alimentation tubulaire débouchant dans la chambre interne, la deuxième portion d'alimentation tubulaire s'étendant autour de la première portion d'alimentation tubulaire. Les première et deuxième portions d'alimentation tubulaires peuvent par exemple s'étendre coaxialement.

Selon un mode de réalisation de l'invention, l'extrémité du troisième conduit d'alimentation tournée vers la chambre de mesure est plus éloignée de l'orifice d'injection que l'extrémité du conduit d'évacuation tournée vers la chambre de mesure.

Selon un mode de réalisation de l'invention, le conduit d'évacuation comporte une portion d'évacuation tubulaire débouchant dans la chambre de mesure, et le troisième conduit d'alimentation comporte une troisième portion d'alimentation tubulaire débouchant reliée fluidiquement à la chambre de mesure, la troisième portion d'alimentation tubulaire s'étendant autour de la portion d'évacuation tubulaire. La portion d'évacuation tubulaire et la troisième portion d'alimentation tubulaire peuvent par exemple s'étendre coaxialement.

Selon un mode de réalisation de l'invention, le premier conduit d'alimentation débouche en regard de l'orifice d'injection.

Selon l'invention, la cellule de mesure est isolée fluidiquement du logement de réception.

Selon un mode de réalisation de l'invention, la cellule de mesure est interposée de manière étanche entre les dispositifs d'injection et d'évacuation.

Selon un mode de réalisation de l'invention, la cellule de mesure est au moins en partie transparente au faisceau lumineux émis par le dispositif d'émission

Selon un mode de réalisation de l'invention, la cellule de mesure est réalisée en un matériau électriquement isolant.

Selon un mode de réalisation de l'invention, les dispositifs d'injection et d'évacuation sont réalisés en un matériau électriquement isolant.

Selon un mode de réalisation de l'invention, le dispositif d'émission est agencé de telle sorte que le chemin optique du faisceau lumineux s'étende sensiblement perpendiculairement à la direction d'écoulement du flux de particules biologiques.

Selon l'invention, le dispositif d'évacuation est monté sur le support à l'opposé du dispositif d'injection par rapport à la cellule de mesure.

Selon l'invention, le support est monobloc.

Selon un mode de réalisation de l'invention, l'au moins un dispositif d'émission et l'au moins un dispositif de collecte s'étendent dans un plan sensiblement perpendiculaire à la direction d'écoulement du flux de particules biologiques.

Selon un mode de réalisation de l'invention, l'ensemble de mesure comporte au moins un élément de détection associé à l'au moins un dispositif de collecte et agencé pour fournir en sortie au moins un signal de mesure déterminé en fonction des rayons lumineux collectés par l'au moins un dispositif de collecte. L'au moins un élément de détection peut par exemple être un photodétecteur, tel qu'une photodiode ou un photomultiplicateur.

Selon un mode de réalisation de l'invention, les particules biologiques à analyser peuvent être des cellules biologiques et notamment des cellules sanguines telles que des leucocytes ou des érythrocytes, ou des plaquettes sanguines, ou encore des levures, des champignons, des spores, des microbes, des bactéries, etc. Les particules biologiques peuvent également être des éléments tels que des cristaux.

Selon un mode de réalisation de l'invention, la buse d'injection comporte un organe d'injection, tel qu'un rubis ou un saphir de synthèse, dans lequel est ménagé l'orifice d'injection. Toutefois, l'orifice d'injection pourrait être également directement moulé dans le corps de la buse d'injection.

Selon un mode de réalisation de l'invention, le dispositif d'émission est disposé de telle sorte que la distance entre l'orifice d'injection et le faisceau lumineux corresponde sensiblement à un tiers ou moins de la distance séparant le conduit d'évacuation et l'orifice d'injection.

Selon un mode de réalisation de l'invention, le dispositif d'émission est disposé de telle sorte que la distance entre l'orifice d'injection et le faisceau lumineux corresponde sensiblement à la moitié ou moins de la hauteur de la chambre de mesure.

Selon un mode de réalisation de l'invention, le premier et/ou le deuxième fluide de gainage est un liquide de dilution, tel qu'un liquide physiologique.

Selon un mode de réalisation de l'invention, le premier et/ou le deuxième fluide de rinçage est un liquide de dilution, tel qu'un liquide physiologique.

La présente invention concerne en outre un ensemble de cytométrie en flux comprenant au moins un système de cytométrie en flux selon l'invention.

Selon un mode de réalisation de l'invention, l'ensemble de cytométrie en flux comprend un boîtier dans lequel est monté l'au moins un système de cytométrie en flux.

Selon un mode de réalisation de l'invention, l'ensemble de cytométrie en flux comprend une unité de pré-amplification agencée pour filtrer et pré-amplifier les signaux de mesure fournis en sortie de l'au moins un élément de détection. Une telle unité de pré-amplification peut par exemple comporter une carte électronique d'acquisition sur laquelle est fixé l'au moins un élément de détection.

Selon un mode de réalisation de l'invention, l'unité de pré-amplification est montée dans le boîtier.

Selon un mode de réalisation de l'invention, l'ensemble de cytométrie en flux comprend un dispositif de contrôle agencé pour contrôler l'ouverture et la fermeture de la première vanne de refoulement et/ou de la deuxième vanne de refoulement.

La présente invention concerne en outre un dispositif d'analyse pour diagnostic in vitro, comprenant au moins un ensemble de cytométrie en flux selon l'invention. Un tel dispositif d'analyse peut par exemple être similaire à celui décrit dans le document FR2998057.

Selon un mode de réalisation de l'invention, le dispositif d'analyse comprend une unité de traitement agencée pour analyser les signaux de mesure fournis par l'au moins un élément de détection. L'unité de traitement est par exemple agencée pour différencier et/ou identifier les particules biologiques, et notamment pour déterminer la structure et/ou la forme des particules biologiques. L'unité de traitement est par exemple également agencée pour déterminer la concentration et/ou la distribution des particules biologiques, et par exemple la concentration et/ou la distribution des leucocytes en lymphocytes, monocytes, neutrophiles, éosinophiles et basophiles.

La présente invention concerne en outre un ensemble comprenant un système de cytométrie en flux selon l'invention et un banc de réglage sur lequel est destiné à être monté le système de cytométrie en flux, dans lequel le banc de réglage comporte au moins un premier dispositif de réglage en translation agencé pour régler en translation la position du dispositif d'émission par rapport au support.

Selon un mode de réalisation de l'invention, le premier dispositif de réglage en translation est agencé pour régler en translation la position du dispositif d'émission par rapport au support selon au moins une première direction de réglage en translation orthogonale à la direction d'écoulement du flux de particules biologiques.

Selon un mode de réalisation de l'invention, le premier dispositif de réglage en translation est agencé pour régler en translation la position du dispositif d'émission par rapport au support selon au moins une deuxième direction de réglage en translation parallèle à la direction d'écoulement du flux de particules biologiques.

Selon un mode de réalisation de l'invention, le premier dispositif de réglage en translation comporte :
- une première partie de fixation fixée sur une partie de support du banc de réglage, telle qu'un plateau de support,
- un élément de support monté mobile en translation par rapport à la première partie de fixation selon la première direction de réglage en translation, et
- une deuxième partie de fixation destiné à être reliée au dispositif d'émission, la deuxième partie de fixation étant montée mobile en translation par rapport à l'élément de support selon la deuxième direction de réglage en translation.

Un tel dispositif réglage en translation permet de régler aisément et avec précision la position du dispositif d'émission, et donc d'assurer un alignement optimal du faisceau lumineux sur le flux de particules biologiques.

Selon un mode de réalisation de l'invention, l'élément de support est une équerre de support comprenant une première et une deuxième branches de support, la première branche de support étant montée mobile en translation par rapport à la première partie de fixation selon la première direction de réglage en translation, la deuxième partie de fixation étant montée mobile en translation par rapport à la deuxième branche de support selon la deuxième direction de réglage en translation.

Selon un mode de réalisation de l'invention, le système de cytométrie en flux comporte une équerre de fixation comprenant une première branche de fixation montée sur le support et sur laquelle est monté le dispositif d'émission, et une deuxième branche de fixation destinée à être fixée sur la deuxième partie de fixation.

Selon un mode de réalisation de l'invention, le coussin de réglage est interposé entre la première partie de montage du dispositif de focalisation et une portion de l'équerre de fixation.

Selon un mode de réalisation de l'invention, le dispositif de réglage en translation est un dispositif de réglage en translation micrométrique.

Selon un mode de réalisation de l'invention, le système de cytométrie en flux comprend au moins une vis de fixation agencée pour fixer la première branche de fixation sur le support, la première branche de fixation comportant au moins un orifice de passage à travers lequel est apte à s'étendre l'au moins une vis de fixation. Selon un mode de réalisation de l'invention, l'orifice de passage est oblong ou présente des dimensions supérieures à celles du corps de la vis de fixation.

Selon un mode de réalisation de l'invention, le banc de réglage comporte au moins un deuxième dispositif de réglage en translation agencé pour régler en translation la position de l'au moins un dispositif de collecte par rapport au support.

Selon un mode de réalisation de l'invention, le deuxième dispositif de réglage en translation est agencé pour régler en translation la position du dispositif de collecte par rapport au support selon au moins une première direction de réglage en translation orthogonale à la direction d'écoulement du flux de particules biologiques.

Selon un mode de réalisation de l'invention, le deuxième dispositif de réglage en translation est agencé pour régler en translation la position du dispositif de collecte par rapport au support selon au moins une deuxième direction de réglage en translation parallèle à la direction d'écoulement du flux de particules biologiques.

Selon un mode de réalisation de l'invention, le deuxième dispositif de réglage en translation comporte :
- une première partie de fixation fixée sur la partie de support du banc de réglage,
- un élément de support monté mobile en translation par rapport à la première partie de fixation selon la première direction de réglage en translation, et
- une deuxième partie de fixation destinée à être reliée au dispositif de collecte, la deuxième partie de fixation étant montée mobile en translation par rapport à l'élément de support selon la deuxième direction de réglage en translation.

Selon un mode de réalisation de l'invention, l'élément de support appartenant au deuxième dispositif de réglage en translation est une équerre de support comprenant une première et une deuxième branches de support, la première branche de support montée mobile en translation par rapport à la première partie de fixation selon la première direction de réglage en translation, la deuxième partie de fixation étant montée mobile en translation par rapport à la deuxième branche de support selon la deuxième direction de réglage en translation.

Selon un mode de réalisation de l'invention, le système de cytométrie en flux comporte au moins une équerre de fixation associée à l'au moins un dispositif de collecte et comprenant une première branche de fixation montée sur le support et sur laquelle est monté le dispositif de collecte, et une deuxième branche de fixation destinée à être fixée sur la deuxième partie de fixation.

Selon un mode de réalisation de l'invention, le deuxième dispositif de réglage en translation est un dispositif de réglage en translation micrométrique.

Selon un mode de réalisation de l'invention, le système de cytométrie en flux comprend au moins une vis de fixation agencée pour fixer sur le support la première branche de fixation sur laquelle est monté le dispositif de collecte, ladite première branche de fixation comportant au moins un orifice de passage à travers lequel est apte à s'étendre l'au moins une vis de fixation. Selon un mode de réalisation de l'invention, l'orifice de passage est oblong ou présente des dimensions supérieures à celles du corps de la vis de fixation correspondante.

De toute façon l'invention sera bien comprise à l'aide de la description qui suit en référence au dessin schématique annexé représentant, à titre d'exemples non limitatifs, deux formes d'exécution de ce système de cytométrie en flux.
Figure 1 est une vue en perspective d'un ensemble de cytométrie en flux comprenant deux systèmes de cytométrie en flux selon un premier mode de réalisation de l'invention.
Figure 2 est une vue en perspective d'un système de cytométrie en flux de la figure 1.
Figure 3 est une vue partielle en perspective du système de cytométrie en flux de la figure 2.
Figure 4 est une vue de dessus du système de cytométrie en flux de la figure 2.
Figure 5 est une vue en coupe selon la ligne V-V de la figure 4.
Figures 6 et 7 sont des vues à l'échelle agrandie de détails de la figure 5.
Figure 8 est une vue en coupe selon la ligne VIII-VIII de la figure 4.
Figures 9 à 11 sont des vues à l'échelle agrandie de détails de la figure 8.
Figure 12 est une vue de face d'une partie d'un dispositif de collecte appartenant au système de cytométrie en flux de la figure 2.
Figures 13 à 15 sont des vues partielles en perspective d'un système de cytométrie en flux installé sur un banc de réglage.
Figures 16 et 17 sont des vues en perspective avant et arrière d'un dispositif d'analyse pour diagnostic in vitro selon l'invention.
Figures 18 et 19 sont des vues en coupe d'un système de cytométrie en flux selon un deuxième mode de réalisation de l'invention.

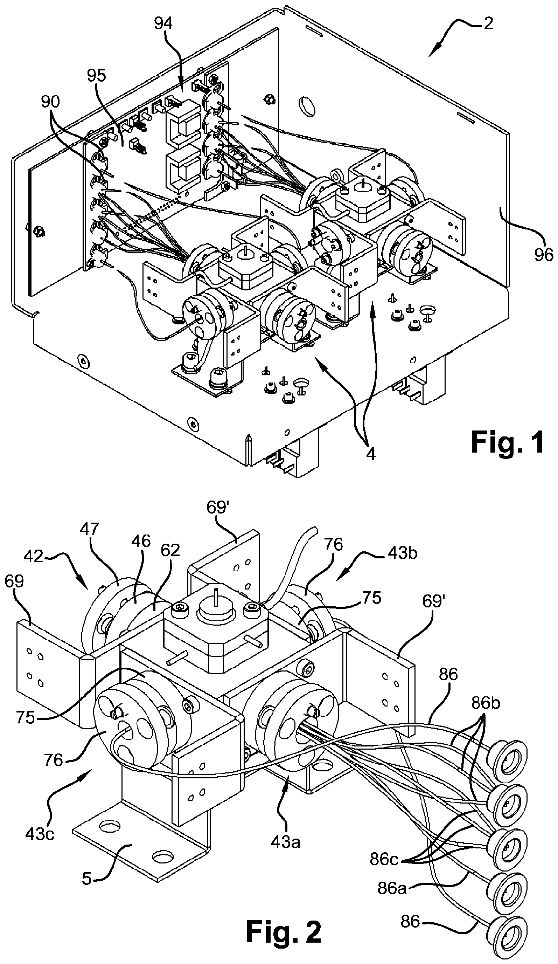

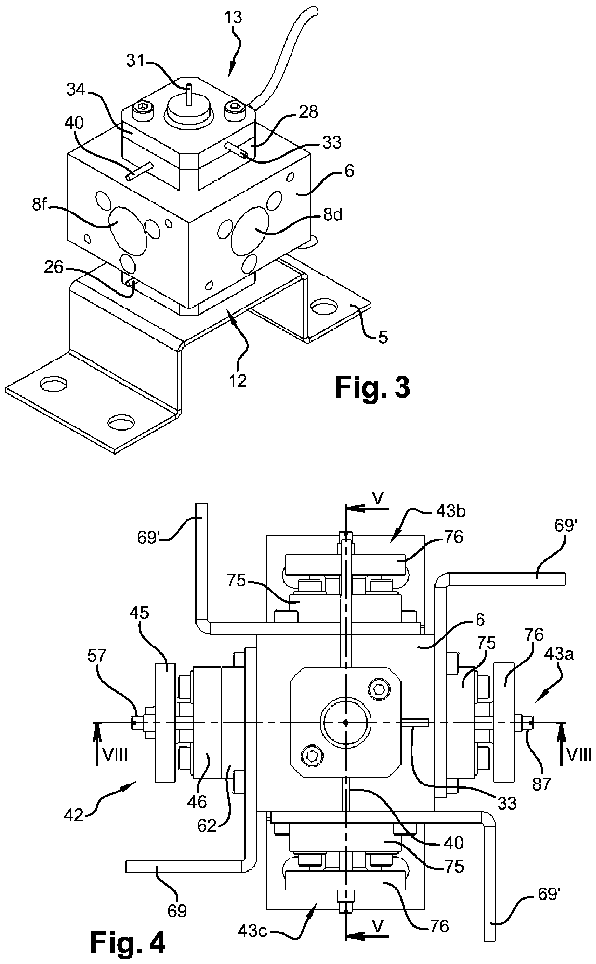

Les figures 1 à 15 représentent un premier mode de réalisation d'un ensemble de cytométrie en flux 2, également nommé ensemble de mesure cytométrique, destiné à l'analyse de particules biologiques, et par exemple de cellules biologiques, telles que des cellules sanguines.

Comme montré sur la figure 1, l'ensemble de cytométrie en flux 2 comprend notamment au moins un système de cytométrie en flux 4, également nommé tête de mesure cytométrique. Selon le mode de réalisation représenté sur la figure 1, l'ensemble de cytométrie en flux 2 comprend deux systèmes de cytométrie en flux 4. Néanmoins, l'ensemble de cytométrie en flux 2 pourrait comprendre un seul système de cytométrie en flux 4 ou plus de deux systèmes de cytométrie en flux 4.

Le système de cytométrie en flux 4 comprend un support 6 monobloc qui peut être par exemple métallique. Le support 6 est parallélépipédique et délimite un logement de réception 7 interne. Le support 6 comporte six ouvertures de passage 8a à 8f ménagées respectivement sur les six faces externes du support 6.

Le système de cytométrie en flux 4 comprend en outre une cellule de mesure 9 qui délimite au moins en partie une chambre de mesure 11, un dispositif d'injection 12 agencé pour injecter un flux de particules biologiques F dans la chambre de mesure 11, et un dispositif d'évacuation 13 agencé pour évacuer à l'extérieur du système de cytométrie en flux 4 le flux de particules biologiques F injecté dans la chambre de mesure 11.

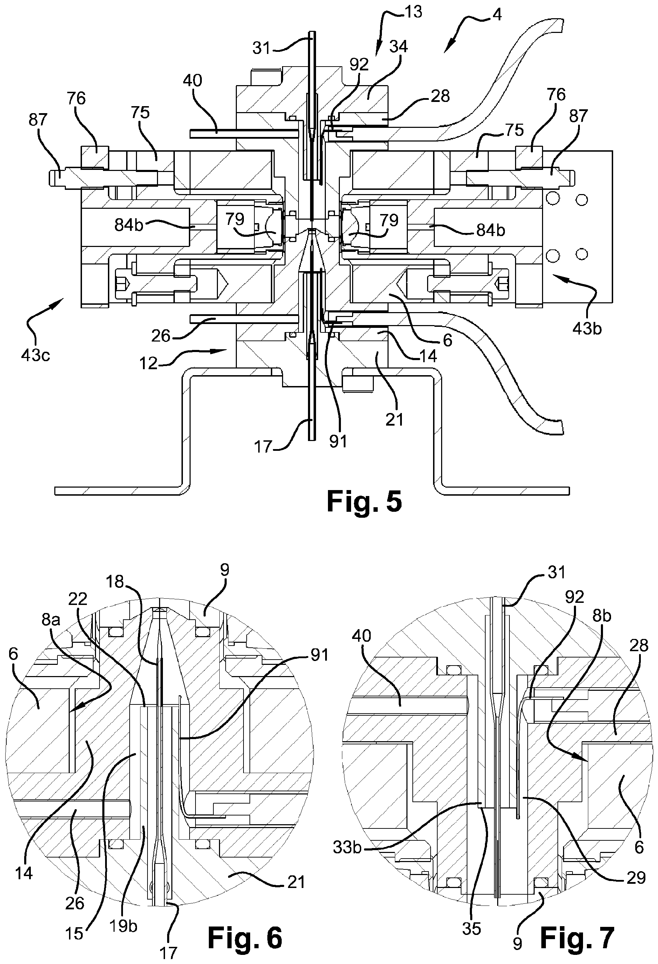

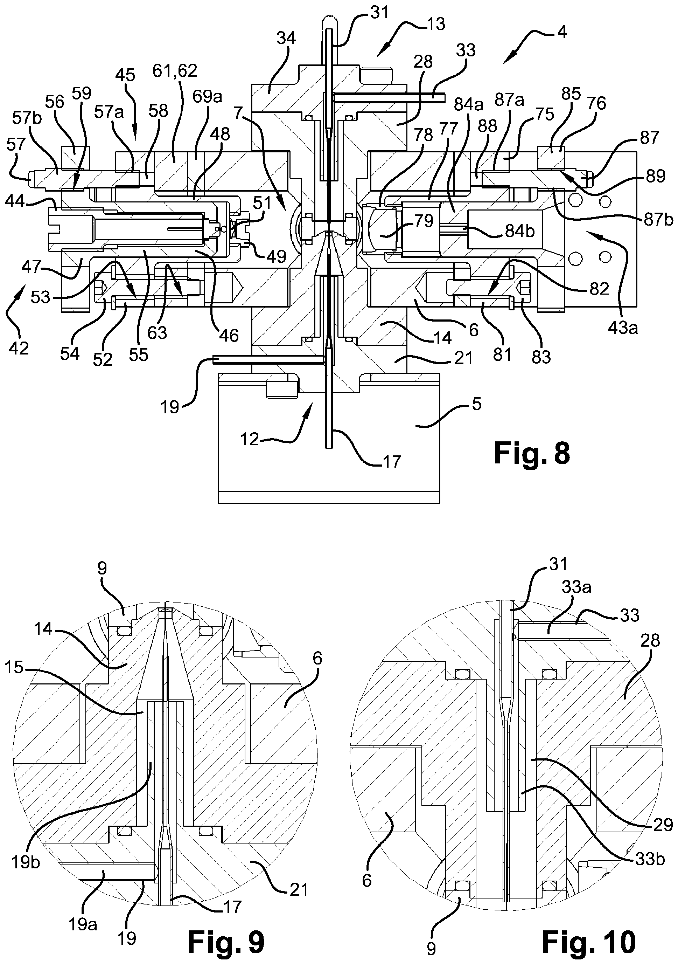

Comme montré sur les figures 5 et 8, la cellule de mesure 9 est annulaire et est interposée de manière étanche entre les dispositifs d'injection et d'évacuation 12, 13. La cellule de mesure 9 est logée dans le logement de réception 7 délimité par le support 5, et est isolée fluidiquement du logement de réception 7. La cellule de mesure 9 est avantageusement réalisée en un matériau électriquement isolant et transparent à la lumière, et par exemple en matière plastique comme du polyméthacrylate de méthyle.

Les dispositifs d'injection et d'évacuation 12, 13 sont fixés respectivement sur deux faces externes opposées du support 6, et par exemple sur les faces externes supérieure et inférieure du support 6.

Comme montré plus particulièrement sur les figures 6 et 9, le dispositif d'injection 12 comprend une buse d'injection 14 délimitant une chambre interne 15. La buse d'injection 14 est munie, à son extrémité supérieure, d'un orifice d'injection 16 débouchant dans la chambre de mesure 11 et agencé pour relier fluidiquement à la chambre interne 15 à la chambre de mesure 11.

Selon le mode de réalisation représenté sur les figures 1 à 15, la buse d'injection 14 comporte d'une part un corps de buse 14a s'étendant en partie à travers l'ouverture de passage 8a du support 6 et réalisé en un matériau électriquement isolant, tel qu'en matière plastique, et d'autre part un organe d'injection 14b monté sur le corps de buse 14a et dans lequel est ménagé l'orifice d'injection 16. L'organe d'injection peut être formé par exemple par un rubis ou un saphir de synthèse, ou encore être réalisé en matière plastique. Selon une variante de réalisation de l'invention, l'orifice d'injection 16 pourrait être injecté directement avec le corps de buse 14a.

Le dispositif d'injection 12 comprend en outre un conduit d'alimentation 17 tubulaire destiné à alimenter la chambre interne 15 avec un échantillon liquide contenant, en suspension, les particules biologiques à analyser. Le conduit d'alimentation 17 s'étend en partie dans la chambre interne 15 et présente une extrémité supérieure 18 débouchant dans la chambre interne 15 à proximité de l'orifice d'injection 16 et en regard de ce dernier.

Le dispositif d'injection 12 comprend en outre un conduit d'alimentation 19 destiné à alimenter la chambre interne 15 avec un fluide de gainage. La buse d'injection 14 et le conduit d'alimentation 19 sont configurés de telle sorte que le fluide de gainage introduit dans la chambre interne 15 via le conduit d'alimentation 19 est apte à gainer hydrodynamiquement l'échantillon liquide introduit dans la chambre interne 15 avant que l'échantillon liquide ne traverse l'orifice d'injection 16. Un tel gainage hydrodynamique peut également être désigné comme une focalisation hydraulique ou hydrodynamique de l'échantillon liquide.

Selon le mode de réalisation représenté sur les figures 1 à 15, le dispositif d'injection 12 comprend une partie d'alimentation 21 montée de manière étanche sur une face inférieure du corps de buse 14a, et le conduit d'alimentation 19 comporte d'une part une première portion de conduit 19a formée par un insert tubulaire monté sur la partie d'alimentation 21, et d'autre part une deuxième portion de conduit 19b tubulaire reliée fluidiquement à la première portion de conduit 19a. La partie d'alimentation 21 peut par exemple être réalisée en un matériau électriquement isolant, et notamment en matière plastique. La deuxième portion de conduit 19b peut par exemple être ménagée sur la partie d'alimentation 21 ou être formée par un insert tubulaire monté sur cette dernière. L'insert tubulaire formant la première portion de conduit 19a peut par exemple être surmoulé.

Selon le mode de réalisation représenté sur les figures 1 à 15, la première portion de conduit 19a comprend une partie d'extrémité faisant saillie de la partie d'alimentation 21 et destinée à être raccordée à une première source de fluide de gainage (non représentée sur les figures). La deuxième portion de conduit 19b s'étend dans la chambre interne 15 et autour du conduit d'alimentation 17, le conduit d'alimentation 17 et la deuxième portion de conduit 19b s'étendant coaxialement. La deuxième portion de conduit 19b présente une extrémité supérieure 22 débouchant dans la chambre interne 15. L'extrémité supérieure 22 de la deuxième portion de conduit 19b est plus éloignée de l'orifice d'injection 16 que l'extrémité supérieure 18 du conduit d'alimentation 17.

Selon le mode de réalisation représenté sur les figures 1 à 15, le conduit d'alimentation 17 comprend une partie d'extrémité s'étendant à travers un orifice de passage ménagé dans la partie d'alimentation 21. La partie d'extrémité du conduit d'alimentation 17 fait saillie de la partie d'alimentation 21 et est destinée à être raccordée à une source d'échantillon liquide (non représentée sur les figures).

Comme montré sur les figures 5 et 6, le dispositif d'injection 12 comporte de plus un conduit de refoulement 26 relié fluidiquement à la chambre interne 15 et destiné à refouler à l'extérieur du système de cytométrie en flux 4 le contenu de la chambre interne 15. Le conduit de refoulement 26 est plus particulièrement destiné à refouler à l'extérieur du système de cytométrie en flux 4 un fluide de rinçage introduit dans la chambre interne 15 via le conduit d'alimentation 19.

Selon le mode de réalisation représenté sur les figures 1 à 15, le conduit de refoulement 26 débouche dans la chambre interne 15, et par exemple à la base de celle-ci, et est formé par un insert tubulaire monté sur le corps de buse 14a et présentant une partie d'extrémité faisant saillie du corps de buse 14a.

L'ensemble de cytométrie en flux 2 comprend en outre une première vanne de refoulement (non représentée sur les figures) reliée fluidiquement au conduit de refoulement 26 et mobile entre une position de fermeture dans laquelle la première vanne de refoulement empêche un écoulement de fluide de la chambre interne 15 vers l'extérieur du système de cytométrie en flux 4 via le conduit de refoulement 26, et une position d'ouverture dans laquelle la première vanne de refoulement autorise un écoulement de fluide de la chambre interne 15 vers l'extérieur du système de cytométrie en flux 4via le conduit de refoulement 26.

Le dispositif d'évacuation 13 comprend une pièce d'évacuation 28 prenant appui contre le support 6 et délimitant une chambre interne 29 débouchant dans la chambre de mesure 11. Une partie de la pièce d'évacuation 28 s'étend à travers l'ouverture de passage 8b du support 6. La pièce d'évacuation 28 peut par exemple être réalisée en un matériau électriquement isolant, et notamment en matière plastique.

Le dispositif d'évacuation 13 comprend en outre un conduit d'évacuation 31 tubulaire relié fluidiquement à la chambre de mesure 11 et destiné à évacuer le flux de particules biologiques F injecté dans la chambre de mesure 11 vers l'extérieur du système de cytométrie en flux 4. Le conduit d'évacuation 31 s'étend en partie dans la chambre interne 29 et présente une extrémité inférieure 32 débouchant dans la chambre de mesure 11 en regard de l'orifice d'injection 16.

Le dispositif d'évacuation 13 comprend de plus un conduit d'alimentation 33 relié fluidiquement à la chambre de mesure 11 et destiné à alimenter la chambre de mesure 11 avec un fluide de gainage. La chambre de mesure 11 et le conduit d'alimentation 33 sont configurés de telle sorte que le fluide de gainage introduit dans la chambre de mesure 11 via le conduit d'alimentation 33 est apte à gainer hydrodynamiquement le flux de particules biologiques F s'écoulant à travers la chambre de mesure 11.

Selon le mode de réalisation représenté sur les figures 1 à 15, le dispositif d'évacuation 13 comprend une partie d'alimentation 34 montée de manière étanche sur une face supérieure de la pièce d'évacuation 28, et le conduit d'alimentation 33 comporte d'une part une première portion de conduit 33a formée par un insert tubulaire monté sur la partie d'alimentation 34, et d'autre part une deuxième portion de conduit 33b tubulaire reliée fluidiquement à la première portion de conduit 33a. La partie d'alimentation 34 peut par exemple être réalisée en un matériau électriquement isolant, et notamment en matière plastique. La deuxième portion de conduit 33b peut par exemple être ménagée sur la partie d'alimentation 34 ou être formée par un insert tubulaire monté sur cette dernière. L'insert tubulaire formant la première portion de conduit 33a peut par exemple être surmoulé.

Selon le mode de réalisation représenté sur les figures 1 à 15, la première portion de conduit 33a comprend une partie d'extrémité faisant saillie de la partie d'alimentation 34 et destinée à être raccordée à une deuxième source de fluide de gainage (non représentée sur les figures). La deuxième portion de conduit 33b s'étend en partie dans la chambre interne 29 et autour du conduit d'évacuation 31, le conduit d'évacuation 31 et la deuxième portion de conduit 33b s'étendant coaxialement. La deuxième portion de conduit 33b présente une extrémité inférieure 35 débouchant dans la chambre interne 29. L'extrémité inférieure 35 de la deuxième portion de conduit 33b est plus éloignée de l'orifice d'injection 16 que l'extrémité inférieure 32 du conduit d'évacuation 31.

Selon un mode de réalisation de l'invention, le système de cytométrie à flux 4 est conformé de telle sorte que la pression du fluide de gainage injecté dans la chambre de mesure 11 via le conduit d'alimentation 33 est inférieure à la pression du fluide de gainage injecté dans la chambre interne 15 via le conduit d'alimentation 26.

Selon le mode de réalisation représenté sur les figures 1 à 15, le conduit d'évacuation 31 comprend une partie d'extrémité s'étendant à travers un orifice de passage ménagée sur la partie d'alimentation 34, et faisant saillie de la partie d'alimentation 34.

Selon un mode de réalisation de l'invention, le corps de buse 14a, la partie d'alimentation 21, la pièce d'évacuation 28 et la partie d'alimentation 34 sont chacun réalisés par moulage.

Comme montré sur les figures 5 et 7, le dispositif d'évacuation 13 comporte de plus un conduit de refoulement 40 relié fluidiquement à la chambre de mesure 11 et destiné à refouler à l'extérieur du système de cytométrie en flux 4 le contenu de la chambre de mesure 11. Le conduit de refoulement 40 est plus particulièrement destiné à refouler à l'extérieur du système de cytométrie en flux 4 un fluide de rinçage introduit dans la chambre de mesure 11 via le conduit d'alimentation 33.

Selon le mode de réalisation représenté sur les figures 1 à 15, le conduit de refoulement 40 débouche dans la chambre interne 29, et est formé par un insert tubulaire monté sur la pièce d'évacuation 28. Le conduit de refoulement 40 est relié fluidiquement à la chambre de mesure 11 via la chambre interne 29.

L'ensemble de cytométrie en flux 2 comprend en outre une deuxième vanne de refoulement (non représentée sur les figures) reliée fluidiquement au conduit de refoulement 40 et mobile entre une position de fermeture dans laquelle la deuxième vanne de refoulement empêche un écoulement de fluide de la chambre de mesure 11 vers l'extérieur du système de cytométrie en flux 4 via le conduit de refoulement 40, et une position d'ouverture dans laquelle la deuxième vanne de refoulement autorise un écoulement de fluide de la chambre de mesure 11 vers l'extérieur du système de cytométrie en flux 4 via le conduit de refoulement 40.

Le système de cytométrie en flux 4 comprend en outre un ensemble de mesure agencé pour mesurer au moins une propriété optique des particules biologiques à analyser.

Selon le mode de réalisation représenté sur les figures 1 à 15, l'ensemble de mesure comporte un dispositif d'émission 42 agencé pour émettre un faisceau lumineux en direction de la chambre de mesure 11 et apte à croiser, c'est-à-dire interséquer, le flux de particules biologiques introduit dans la chambre de mesure 11, et plusieurs dispositifs de collecte 43a, 43b, 43c décalés angulairement par rapport au flux de particules biologiques et agencés pour collecter des rayons lumineux issus de la chambre de mesure 11. Néanmoins, l'ensemble de mesure pourrait comporter par exemple plusieurs dispositifs d'émission décalés angulairement par rapport au flux de particules biologiques, et également uniquement un seul ou plusieurs dispositifs de collecte.

Les dispositifs d'émission et de collecte sont montés sur les faces latérales du support 6 et s'étendent dans un plan sensiblement perpendiculaire à la direction d'écoulement du flux de particules biologiques F. Le dispositif de collecte 43a est par exemple disposé à l'opposé du dispositif d'émission 42 par rapport à la cellule de mesure 9, tandis que les dispositifs de collecte 43b et 43c sont disposés perpendiculairement au dispositif d'émission 42 par rapport à la cellule de mesure 9. Les dispositifs d'émission et de collecte 43a-43c s'étendent respectivement en partie à travers les ouvertures de passage 8c à 8f du support 6.

Le dispositif d'émission 42 comprend une source lumineuse 44 agencée pour générer le faisceau lumineux. La source lumineuse 44 peut être par exemple une source laser agencée pour générer un faisceau laser.

Le dispositif d'émission 42 comporte un dispositif de focalisation 45 agencé pour focaliser le faisceau lumineux émis par la source lumineuse 44, dans la chambre de mesure 11 et sur le flux de particules biologiques F.

Selon le mode de réalisation représenté sur les figures 1 à 15, le dispositif de focalisation 45 comprend une première partie de montage 46 destinée à être immobilisée par rapport au support 6, et une deuxième partie de montage 47 sur laquelle est montée la source lumineuse 44. La deuxième partie de montage 47 est montée mobile en translation par rapport à la première partie de montage 46 selon une direction de déplacement D1 parallèle au chemin optique du faisceau lumineux.

Comme montré sur la figure 8, la première partie de montage 46 comprend une portion de guidage 48 tubulaire délimitant un conduit de guidage. La portion de guidage 48 est équipée d'un élément optique de focalisation 49 disposé sur le chemin optique du faisceau lumineux. L'élément optique de focalisation 49 comprend par exemple une lentille de focalisation 51.

La première partie de montage 46 comprend également une portion d'appui 52 annulaire s'étendant radialement à partir de la portion de guidage 48. La portion d'appui 52 comprend une pluralité d'orifices de passage 53 destinés au passage de vis d'immobilisation 54 agencées pour immobilier la première partie de montage 46 par rapport au support 6. Les orifices de passage 53 sont par exemple décalés angulairement par rapport à l'axe d'extension de la première partie de montage 46. Selon le mode de réalisation représenté sur les figures 1 à 15, la première partie de montage 46 comprend trois orifices de passage 53 régulièrement décalés angulairement, et trois vis d'immobilisation 54.

La deuxième partie de montage 47 comprend une portion guidée 55 tubulaire montée coulissante dans le conduit de guidage délimité par la première partie de montage 46. La portion guidée 55 délimite un logement dans lequel est montée la source lumineuse 44. La portion guidée 55 comprend par exemple une ouverture disposée en regard de l'élément optique de focalisation 49 et à travers laquelle s'étend une portion d'émission de la source lumineuse 44.

La deuxième partie de montage 47 comprend en outre une portion annulaire 56 s'étendant radialement à partir de la portion guidée 55.

Le dispositif de focalisation 45 comprend en outre un élément de réglage micrométrique 57 agencé pour régler la position relative des première et deuxième parties de montage le long de la direction de déplacement D1. Selon le mode de réalisation représenté sur les figures 1 à 15, l'élément de réglage micrométrique 57 comprend une première portion filetée 57a agencée pour coopérer avec un premier alésage taraudé 58 ménagé sur la première partie de montage 46 du dispositif de focalisation, et une deuxième portion filetée 57b agencée pour coopérer avec un deuxième alésage taraudé 59 ménagé sur la deuxième partie de montage 47 du dispositif de focalisation, les première et deuxième portions filetées 57a, 57b présentant des filetages de pas différents.

Le système de cytométrie en flux 4 comporte de plus un dispositif de réglage d'orientation 61, également nommé dispositif de réglage d'assiette, agencé pour régler l'orientation ou l'assiette du faisceau lumineux émis par le dispositif d'émission de telle sorte que le chemin optique du faisceau lumineux s'étende sensiblement orthogonalement à la direction d'écoulement du flux de particules biologiques F.

Le dispositif de réglage d'orientation 61 comporte un coussin de réglage 62 annulaire disposé entre le support 6 et la portion d'appui 52 de la première partie de montage 46 du dispositif de focalisation 45. Le coussin de réglage 62 est au moins en partie élastiquement déformable

Selon le mode de réalisation représenté sur les figures 1 à 15, le coussin de réglage 62 délimite un passage central à travers lequel s'étend la portion de guidage 48 de la première partie de montage 46, et comporte une pluralité d'orifices 63 de passage à travers lesquels s'étendent les vis d'immobilisation 54.

Une telle disposition et une telle configuration du coussin de réglage 62 permettent à un opérateur de régler aisément l'assiette du faisceau lumineux émis par la source lumineuse 44 tout simplement en vissant et/ou en dévissant les différentes vis d'immobilisation 54 qui provoquent une déformation élastique du coussin de réglage 62.

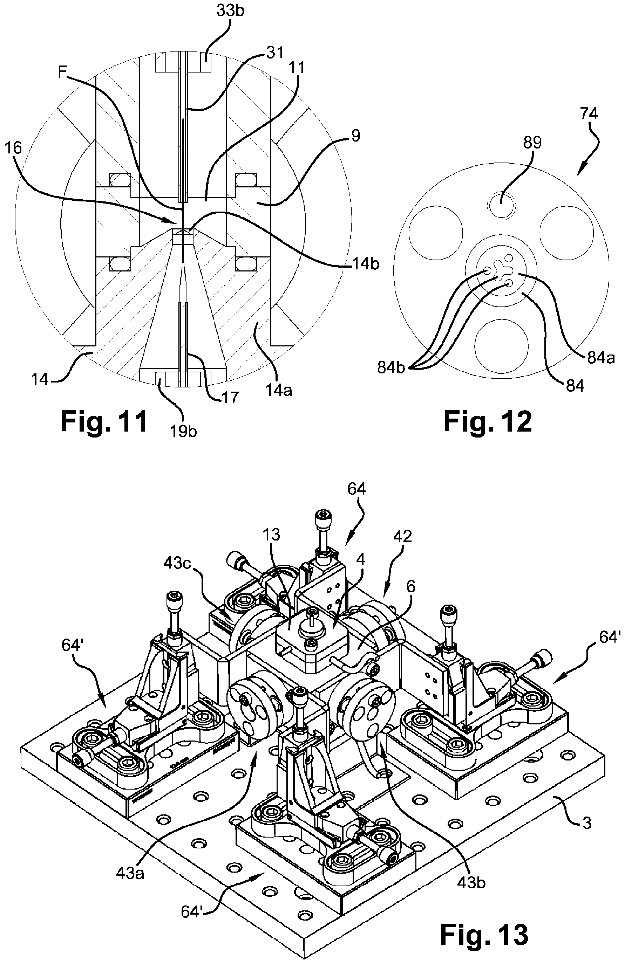

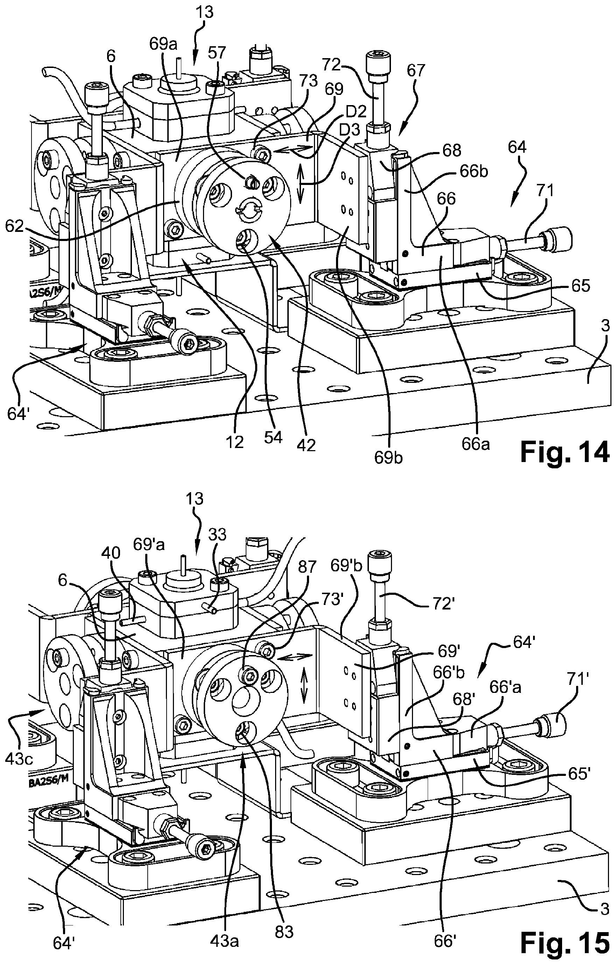

Comme montré plus particulièrement sur les figures 13 et 15, le système de cytométrie en flux 4 peut être fixé sur un banc de réglage 3 à l'aide d'une embase 5 en vue de réaliser un réglage en position du dispositif d'émission 42. Le banc de réglage 3 comporte un dispositif de réglage en translation 64 agencé pour régler en translation la position du dispositif d'émission 42 par rapport au support 6 selon une première direction de réglage en translation D2 orthogonale à la direction d'écoulement du flux de particules biologiques F et une deuxième direction de réglage en translation D3 parallèle à la direction d'écoulement du flux de particules biologiques F.

Le premier dispositif de réglage en translation 64 comporte une première partie de fixation 65 fixée sur un plateau du banc de réglage 3. Le premier dispositif de réglage en translation 64 comporte en outre une équerre de support 66 comprenant une première et une deuxième branches de support 66a, 66b perpendiculaires l'une par rapport à l'autre. La première branche de support 66a est montée mobile en translation sur la première partie de fixation 65 selon la première direction de réglage en translation D2.

Le premier dispositif de réglage en translation 64 comporte également une deuxième partie de fixation 67 sur laquelle est destiné à être monté le dispositif d'émission 42. La deuxième partie de fixation 67 comprend une pièce de fixation 68 montée mobile en translation sur la deuxième branche de support 66b de l'équerre de support 66 selon la deuxième direction de réglage en translation D2.

Le dispositif de réglage en translation 64 comprend également un vis micrométrique 71 agencée pour régler la position de l'équerre de support 66 par rapport à la première partie de fixation 65, et une vis micrométrique 72 agencée pour régler la position de la pièce de fixation 68 par rapport à l'équerre de support 66.

Le système de cytométrie en flux 4 comprend en outre une équerre de fixation 69 comprenant une première branche de fixation 69a fixée sur le support 6 et sur laquelle est monté le dispositif d'émission 42, et une deuxième branche de fixation 69b destinée à être fixée sur la pièce de fixation 68.

Le système de cytométrie en flux 4 comprend également une pluralité de vis de fixation 73 agencées pour fixer la première branche de fixation 69a de l'équerre de fixation 69 sur le support 6, et la première branche de fixation 69a comporte une pluralité d'orifices de passage 74 à travers lesquels s'étendent les vis de fixation 73. Selon le mode de réalisation représenté sur les figures 1 à 15, chaque orifice de passage 74 présente des dimensions supérieures à celles du corps de la vis de fixation 73 correspondante.

Afin de régler précisément la position du dispositif d'émission 42 par rapport au support 6 et donc d'assurer un croisement optimal du faisceau lumineux et du flux de particules biologiques F dans la chambre de mesure 11, un opérateur doit tout d'abord installer le système de cytométrie en flux 4 sur le banc de réglage 3 et fixer la deuxième branche de fixation 69b sur la pièce de fixation 68, puis desserrer les vis de fixation 74, ensuite actionner d'une part la vis micrométrique 71 de manière à régler horizontalement la position du faisceau lumineux et d'autre part la vis micrométrique 72 de manière à régler verticalement la position du faisceau lumineux, et enfin resserrer les vis de fixation 74 de manière à immobiliser l'équerre de fixation 69 par rapport au support 6. Le dispositif de réglage en translation 64 permet donc un réglage en translation aisé de la position du dispositif d'émission 42.

Selon le mode de réalisation représenté sur les figures 1 à 15, le coussin de réglage 62 est interposé entre la première branche de fixation 69a de l'équerre de fixation 69 et la première partie de montage 46 du dispositif de focalisation 45.

Comme montré sur les figures 5 et 8, chaque dispositif de collecte 43a, 43b, 43c comporte une première partie de montage 75 destinée à être immobilisée par rapport au support 6, et une deuxième partie de montage 76 montée mobile en translation par rapport à la première partie de montage 75 selon la direction de déplacement.

Selon le mode de réalisation représenté sur les figures 1 à 15, la première partie de montage 75 de chaque dispositif de collecte comprend une portion de guidage 77 tubulaire délimitant un conduit de guidage. La portion de guidage 77 est équipée d'un élément optique de collecte 78 disposé à proximité de la cellule de mesure 9. L'élément optique de collecte 78 comprend par exemple une lentille optique 79.

La première partie de montage 75 de chaque dispositif de collecte comprend également une portion d'appui 81 annulaire s'étendant radialement à partir de la portion de guidage 77 correspondante. Chaque portion d'appui 81 comprend une pluralité d'orifices de passage 82 destinés au passage de vis d'immobilisation 83 agencées pour immobilier la première partie de montage 75 correspondante par rapport au support 6. Les orifices de passage 83 prévus sur chaque portion d'appui 81 sont par exemple décalés angulairement par rapport à l'axe d'extension de la première partie de montage 75 correspondante. Selon le mode de réalisation représenté sur les figures 1 à 15, chaque première partie de montage 75 comprend trois orifices de passage 82 régulièrement décalés angulairement, et trois vis d'immobilisation 83.

La deuxième partie de montage 76 comprend une portion guidée 84 tubulaire montée coulissante dans le conduit de guidage délimité par la première partie de montage 75, et une portion annulaire 85 s'étendant radialement à partir de la portion guidée 84. La portion guidée 84 comporte une paroi d'extrémité 84a tournée vers la cellule de mesure 9 et dans laquelle est ménagé au moins un orifice de montage 84b dans lequel est montée une fibre optique de collecte 86.

Chaque dispositif de collecte 43a-43c comprend en outre un élément de réglage micrométrique 87 agencé pour régler la position relative des première et deuxième parties de montage 75, 76 du dispositif de collecte correspondant le long de la direction de déplacement correspondante. Selon le mode de réalisation représenté sur les figures 1 à 15, chaque élément de réglage micrométrique 87 comprend une première portion filetée 87a agencée pour coopérer avec un premier alésage taraudé 88 ménagé sur la première partie de montage 75 correspondante, et une deuxième portion filetée 87b agencée pour coopérer avec un deuxième alésage taraudé 89 ménagé sur la deuxième partie de montage 76 correspondante, les première et deuxième portions filetées 87a, 87b présentant des filetages de pas différents.

Selon le mode de réalisation représenté sur les figures 1 à 15 et comme cela découle plus particulièrement de la figure 2, le dispositif de collecte 43a comprend une pluralité de fibres optiques de collecte, et plus particulièrement une fibre optique de collecte centrale 86a, et des fibres optiques de collecte périphériques 86b, 86c. Par exemple, la fibre optique de collecte centrale 86a est destinée à collecter les rayons lumineux issus de la chambre de mesure 11 selon le chemin optique du faisceau lumineux incident, c'est-à-dire à 0°, au moins une fibre optique de collecte périphérique 86b est destinée à collecter des rayons lumineux issus de la chambre de mesure 11 à un angle de l'ordre de 4° et au moins une fibre optique de collecte périphérique 86c est destinée à collecter des rayons lumineux issus de la chambre de mesure 11 à un angle de l'ordre de 9°. Le dispositif de collecte 43a peut par exemple comprend plusieurs fibres optiques de collecte périphériques 86b destinées à collecter des rayons lumineux issus de la chambre de mesure 11 à un angle de l'ordre de 4° et plusieurs fibres optiques de collecte périphériques 86c destinées à collecter des rayons lumineux issus de la chambre de mesure 11 à un angle de l'ordre de 9°.

Selon le mode de réalisation représenté sur les figures 1 à 15, chacun des dispositifs de collecte 43b, 43c comprend une seule fibre optique de collecte centrale.

Le banc de réglage 3 comporte en outre trois dispositifs de réglage en translation 64' destinés à être associés chacun à l'un des dispositifs de collecte 43a-43c. Selon le mode de réalisation représenté sur les figures 1 à 15, les dispositifs de réglage en translation 64' sont identiques au dispositif de réglage 64 destiné à être associé au dispositif d'émission 42.

Chaque dispositif de réglage en translation 64' comporte une première partie de fixation 65' fixée sur le plateau du banc de réglage 3 et une équerre de support 66' comprenant une première et une deuxième branches de support 66a', 66b' perpendiculaires l'une par rapport à l'autre. La première branche de support 66a' de chaque équerre de support 66' est montée mobile en translation sur la première partie de fixation 65' correspondante selon la première direction de réglage en translation orthogonale à la direction d'écoulement du flux de particules biologiques F.

Chaque dispositif de réglage en translation 64' comporte également une deuxième partie de fixation 67' sur laquelle est destiné à être monté le dispositif de collecte correspondant. La deuxième partie de fixation 67' de chaque dispositif de réglage en translation 64' comprend une pièce de fixation 68' montée mobile en translation sur la deuxième branche de support 66b' de l'équerre de support 66' correspondante selon une deuxième direction de réglage en translation parallèle à la direction d'écoulement du flux de particules biologiques F.

Chaque dispositif de réglage en translation 64' comprend également un vis micrométrique 71' agencée pour régler la position de l'équerre de support 66' dudit dispositif de réglage en translation 64' par rapport à la première partie de fixation 65' correspondante, et une vis micrométrique 72 agencée pour régler la position de la pièce de fixation 68' dudit dispositif de réglage en translation 64' par rapport à l'équerre de support 66' correspondante.

Le système de cytométrie en flux 4comprend en outre une équerre de fixation 69' associée à chaque dispositif de collecte. Chaque équerre de fixation 69' comprend une première branche de fixation 69a' fixée sur le support 6 et sur laquelle est monté le dispositif de collecte correspondant 42, et une deuxième branche de fixation 69b' destinée à être fixée sur la pièce de fixation 68' correspondante.

Le système de cytométrie en flux 4 comprend également une pluralité de vis de fixation 73' agencées pour fixer la première branche de fixation 69a' de chaque équerre de fixation 69' sur le support 6, et chaque première branche de fixation 69a' comporte une pluralité d'orifices de passage 74' à travers lesquels s'étendent les vis de fixation 73' correspondantes. Selon le mode de réalisation représenté sur les figures 1 à 15, chaque orifice de passage 74' présente des dimensions supérieures à celles du corps de la vis de fixation 73' correspondante.

Afin de régler précisément la position de chaque dispositif de collecte 43a-43c par rapport au support 6 et donc d'assurer une collecte optimale des rayons lumineux issus de la chambre de mesure 11, un opérateur doit tout d'abord installer le système de cytométrie en flux 4 sur le banc de réglage 3 et fixer les deuxièmes branches de fixation 69b' sur les pièces de fixation 68' respectives, puis desserrer les vis de fixation 74' associées à chaque dispositif de réglage en translation 64', ensuite actionner d'une part les vis micrométriques 71' de manière à régler horizontalement la position des différents dispositifs de collecte et d'autre part les vis micrométriques 72 de manière à régler verticalement la position des différents dispositifs de collecte, et enfin resserrer les vis de fixation 74' de manière à immobiliser les différentes équerres de fixation 69' par rapport au support 6. Chaque dispositif de réglage en translation 64' permet donc un réglage en translation aisé de la position du dispositif de collecte 43a-43c correspondant.

L'ensemble de mesure comporte en outre une pluralité d'éléments de détection 90 associés chacun à un dispositif de collecte 43a-43c. Chaque élément de détection 90 est agencé pour fournir en sortie un signal de mesure déterminé en fonction des rayons lumineux collectés par le dispositif de collecte correspondant. Au passage de chaque particule biologique à travers le faisceau lumineux incident, chaque signal de mesure fourni en sortie par chaque élément de détection 90 est par exemple proportionnel à la quantité de lumière absorbée ou réémise par ladite particule biologique. Chaque élément de détection 90 peut par exemple être un photodétecteur, tel qu'une photodiode ou également photomultiplicateur.

L'ensemble de cytométrie en flux 2 comprend en outre une unité de pré-amplification 94 agencée pour filtrer et pré-amplifier les signaux de mesure fournis en sortie des différents éléments de détection 90. L'unité de pré-amplification 94 comporte notamment une carte électronique d'acquisition 95 sur laquelle sont fixés les éléments de détection 90.

L'ensemble de cytométrie en flux 2 comprend également un boîtier 96 dans lequel sont logés notamment chaque système de cytométrie en flux 4, les éléments de détection 90 et l'unité de pré-amplification 94.

Le système de cytométrie en flux 4 comprend en outre un dispositif de mesure de variation d'impédance électrique agencé pour mesurer la variation d'impédance électrique générée par le passage des particules biologiques à travers l'orifice d'injection 16. Le dispositif de mesure de variation d'impédance électrique comprend par exemple une première et une deuxième électrodes 91, 92 disposées respectivement de part et d'autre de l'orifice d'injection 16. Les première et deuxième électrodes 91, 92 sont destinées à être en contact électrique avec le flux de particules biologiques F de manière à établir un champ électrique au travers de l'orifice d'injection 16. Un tel dispositif de mesure de variation d'impédance électrique permet de compter le nombre de particules biologiques passant par l'orifice d'injection 16, et également de déterminer la taille, et plus précisément le volume des particules biologiques. Le fonctionnement d'un tel dispositif de mesure de variation d'impédance électrique est connu de l'homme du métier et n'est donc pas décrit en détails. Il doit cependant être noté que le passage de chaque particule biologique à travers l'orifice d'injection 16 provoque une impulsion électrique proportionnelle à la taille ou volume de ladite particule biologique et permettant de compter électriquement le nombre de particules.

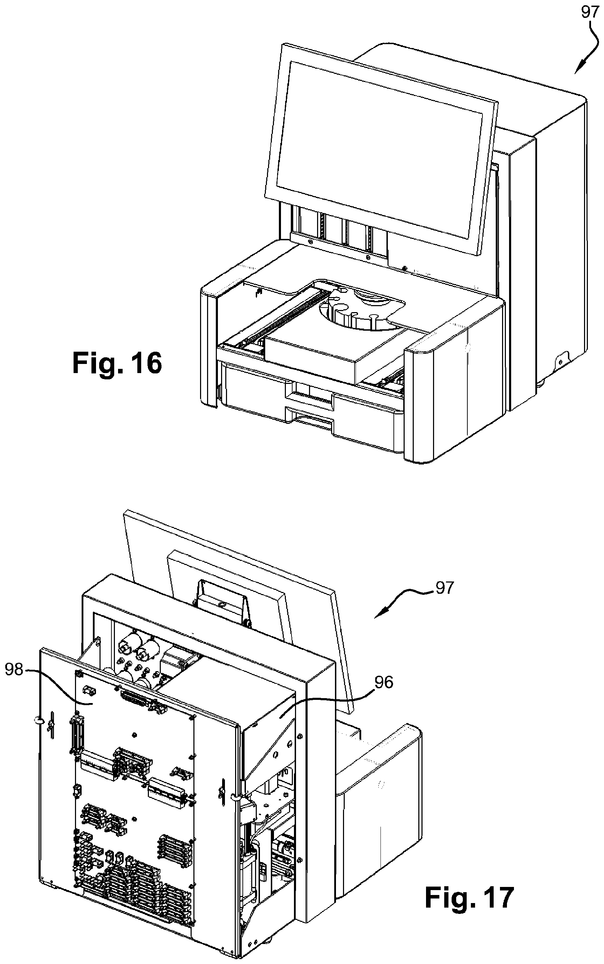

Les figures 16 et 17 représentent un dispositif d'analyse 97 pour diagnostic in vitro, et par exemple pour réaliser des tests sanguins, tels que de tests sur sang total. Un tel dispositif d'analyse 97 comprend notamment un ensemble de cytométrie en flux 2 et une unité de traitement 98 agencée pour analyser les signaux de mesure fournis par chaque élément de détection 90.

L'unité de traitement 98 est par exemple agencée pour différencier et/ou identifier les particules biologiques, et notamment pour déterminer la structure et/ou la forme des particules biologiques à partir des signaux de mesure fournis par les éléments de détection 90. L'unité de traitement 98 peut également être agencée pour déterminer la concentration et/ou la distribution des particules biologiques. Une telle unité de traitement 98 est connue de l'homme du métier et n'est donc pas décrite en détails.

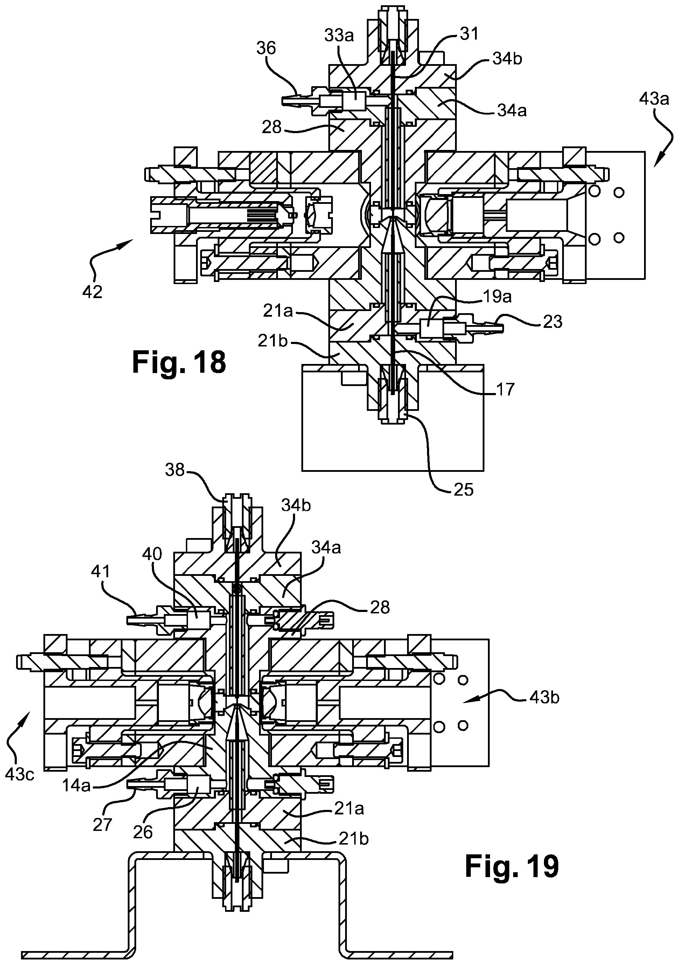

Les figures 18 et 19 représentent un système de cytométrie en flux 4 selon un deuxième mode de réalisation de l'invention qui diffère de celle représentée sur les figures 1 à 15 essentiellement en ce que la partie d'alimentation 21 est formée par une première et deuxième pièces d'alimentation 21a, 21b distinctes l'une de l'autre, en ce que la partie d'alimentation 34 est formée par une première et deuxième pièce d'alimentation 34a, 34b distinctes l'une de l'autre, en ce que la première portion de conduit 19a est ménagée sur la première pièce d'alimentation 21a, en ce que le conduit de refoulement 26 est ménagé sur le corps de buse 14a, en ce que la première portion de conduit 33a est ménagée sur la première partie d'alimentation 34a et en ce que le conduit de refoulement 40 est ménagé sur la pièce d'évacuation 28.

Selon ce mode de réalisation de l'invention, le dispositif d'injection 12 comporte un premier embout de raccordement 23 relié fluidiquement à la première portion de conduit 19a et monté sur la première partie d'alimentation 21a, un deuxième embout de raccordement 25 destiné à être raccordé à la source d'échantillon liquide et monté sur la deuxième partie d'alimentation 21b, et un troisième embout de raccordement 27 relié fluidiquement au conduit de refoulement 26 et monté sur le corps de buse 14a. Selon ce mode de réalisation de l'invention, le dispositif d'évacuation 13 comprend un premier embout de raccordement 36 destiné à être relié à la deuxième source de fluide de gainage et monté sur la première partie d'alimentation 34a, un deuxième embout de raccordement 38 relié fluidiquement au conduit d'évacuation 31 et monté sur la deuxième partie d'alimentation 34b, et un troisième embout de raccordement 41 monté sur la pièce d'évacuation 28 et relié fluidiquement au conduit de refoulement 40.

Selon un autre mode de réalisation de l'invention non représenté sur les figures, les première et deuxième électrodes du dispositif de mesure de variation d'impédance électrique pourraient être formées par les conduits d'alimentation et d'évacuation 17, 31 ou encore par les portions de conduit 19b, 33b.

Selon un autre mode de réalisation de l'invention non représenté sur les figures, l'ensemble de mesure pourrait comporter deux dispositifs d'émission 42 décalés angulairement, et deux séries de dispositifs de collecte chacune associée à l'un des dispositifs d'émission 42, chaque série comportant par exemple trois dispositifs de collecte 43a-43c décalés angulairement. Selon un tel mode de réalisation, le support 6 peut par exemple présenter une forme octogonale. Selon un tel mode de réalisation, les dispositifs d'émission 42 peuvent présenter des sources lumineuses différentes. Par exemple, l'un des dispositifs d'émission 42 pourrait être agencé pour émettre un faisceau laser bleu et l'autre dispositif d'émission 42 pourrait être agencé pour émettre un faisceau laser rouge.

Comme il va de soi, l'invention ne se limite pas aux seules formes d'exécution de ce système de cytométrie en flux, décrites ci-dessus à titre d'exemples, elle en embrasse au contraire toutes les variantes de réalisation entrant dans le cadre des revendications.

## Revendications

1. Système de cytométrie en flux (4) comprenant :
- une cellule de mesure (9) délimitant au moins en partie une chambre de mesure (11),
- un dispositif d'injection (12) agencé pour injecter un flux de particules biologiques à analyser dans la chambre de mesure (11), le dispositif d'injection (12) comprenant :
- une buse d'injection (14) délimitant une chambre interne (15) et comprenant un orifice d'injection (16) relié fluidiquement à la chambre de mesure (11),
- un premier conduit d'alimentation (17) débouchant dans la chambre interne (15) et destiné à alimenter la chambre interne avec un échantillon liquide contenant les particules biologiques à analyser, et
- un deuxième conduit d'alimentation (19) débouchant dans la chambre interne (15) et destiné à alimenter la chambre interne avec un premier fluide de gainage, la buse d'injection (14) et le deuxième conduit d'alimentation (19) étant configurés de telle sorte que le premier fluide de gainage introduit dans la chambre interne est apte à gainer hydrodynamiquement l'échantillon liquide introduit dans la chambre interne,
- un dispositif d'évacuation (13) agencé pour évacuer à l'extérieur du système de cytométrie le flux de particules biologiques injecté dans la chambre de mesure (11),
- un troisième conduit d'alimentation (33) relié fluidiquement à la chambre de mesure (11) et destiné à alimenter la chambre de mesure avec un deuxième fluide de gainage, la chambre de mesure (11) et le troisième conduit d'alimentation (33) étant configurés de telle sorte que le deuxième fluide de gainage introduit dans la chambre de mesure (11) est apte à gainer hydrodynamiquement le flux de particules biologiques dans la chambre de mesure (11),
- un ensemble de mesure agencé pour mesurer au moins une propriété optique des particules biologiques à analyser, l'ensemble de mesure comportant :
- au moins un dispositif d'émission (42) agencé pour émettre un faisceau lumineux en direction de la chambre de mesure (11) et apte à croiser le flux de particules biologiques, l'au moins un dispositif d'émission (42) comprenant une source lumineuse (44) agencée pour générer le faisceau lumineux,
- au moins un dispositif de collecte (43a-43c) agencé pour collecter des rayons lumineux issus de la chambre de mesure (11),
**caractérisé en ce que** le système de cytométrie en flux comprend en outre un support de référence (6) qui est monobloc et sur lequel sont montés le dispositif d'injection (12), le dispositif d'évacuation (13), l'au moins un dispositif d'émission (42) et l'au moins un dispositif de collecte (43a-43c), **en ce que** le support de référence (6) délimite un logement de réception (7) dans lequel est logée la cellule de mesure (9), la cellule de mesure (9) étant isolée fluidiquement du logement de réception (7), et **en ce que** les dispositifs d'injection et d'évacuation (12, 13) sont fixés respectivement sur deux faces externes opposées du support de référence (6), le dispositif d'évacuation (13) étant monté sur le support de référence (6) à l'opposé du dispositif d'injection (12) par rapport à la cellule de mesure (9).

2. Système de cytométrie en flux selon la revendication 1, dans lequel l'au moins un dispositif d'émission (42) comporte un dispositif de focalisation (45) agencé pour focaliser le faisceau lumineux dans la chambre de mesure (11) et sur le flux de particules biologiques (F).

3. Système de cytométrie en flux selon la revendication 2, dans lequel le dispositif de focalisation (45) comprend :
- une première partie de montage (46) équipée d'un élément optique de focalisation (49) disposé sur le chemin optique du faisceau lumineux,
- une deuxième partie de montage (47) sur laquelle est montée la source lumineuse (44), les première et deuxième parties de montage (46, 47) du dispositif de focalisation (45) étant déplaçables l'une par rapport à l'autre selon une première direction de déplacement (D1) sensiblement parallèle au chemin optique du faisceau lumineux, et
- un premier élément de réglage (57) agencé pour régler la position relative des première et deuxième parties de montage (46, 47) du dispositif de focalisation (45) le long de la première direction de déplacement (D1).

4. Système de cytométrie en flux selon la revendication 3, dans lequel le dispositif de focalisation (45) comprend au moins un élément d'immobilisation (54) agencé pour immobilier la première partie de montage (46) par rapport au support de référence (6), la deuxième partie de montage (46) du dispositif de focalisation (45) étant monté mobile par rapport à la première partie de montage (46) du dispositif de focalisation (45).

5. Système de cytométrie en flux selon l'une quelconque des revendications 1 à 4, lequel comporte un dispositif de réglage d'orientation (61) agencé pour régler l'orientation du faisceau lumineux émis par l'au moins un dispositif d'émission (42).

6. Système de cytométrie en flux selon la revendication 5, dans lequel le dispositif de réglage d'orientation (61) comporte :
- un coussin de réglage (62) disposé entre le support de référence (6) et l'au moins un dispositif d'émission (42), le coussin de réglage (62) étant au moins en partie élastiquement déformable, et
- un ensemble de déformation agencé pour déformer le coussin de réglage (62) de manière à régler l'orientation du faisceau lumineux émis par l'au moins un dispositif d'émission (42).

7. Système de cytométrie en flux selon la revendication 6 en combinaison avec la revendication 3, dans lequel la première portion de montage (46) du dispositif de focalisation (45) comporte une portion d'appui (52) agencée pour prendre appui contre le coussin de réglage (62).

8. Système de cytométrie en flux selon la revendication 7 en combinaison avec la revendication 4, dans lequel l'ensemble de déformation est formé par l'au moins un élément d'immobilisation et la portion d'appui (52) de la première portion de montage (46).

9. Système de cytométrie en flux selon l'une quelconque des revendications 1 à 8, dans lequel l'au moins un dispositif de collecte (43a-c) comporte :
- une première partie de montage (75) comprenant un premier élément optique de collecte (78),
- une deuxième partie de montage (76) comprenant au moins un deuxième élément optique de collecte (86), les première et deuxième parties de montage (75, 76) de l'au moins un dispositif de collecte étant déplaçables l'une par rapport à l'autre selon une deuxième direction de déplacement, et
- un deuxième élément de réglage (87) agencé pour régler la position relative des première et deuxième parties de montage (75, 76) de l'au moins un dispositif de collecte (43a-43c) le long de la deuxième direction de déplacement.

10. Système de cytométrie en flux selon la revendication 9, dans lequel l'au moins un dispositif de collecte (43a-43c) comprend au moins un élément d'immobilisation (83) agencé pour immobilier la première partie de montage (75) dudit dispositif de collecte par rapport au support de référence (6), la deuxième partie de montage (76) dudit dispositif de collecte étant monté mobile par rapport à la première partie de montage dudit dispositif de collecte.

11. Système de cytométrie en flux selon l'une quelconque des revendications 8 à 10, dans lequel l'au moins un deuxième élément optique de collecte (86) comprend au moins une fibre optique de collecte.

12. Système de cytométrie en flux selon l'une quelconque des revendications 1 à 11, lequel comprend un dispositif de mesure de variation d'impédance électrique agencé pour mesurer une variation d'impédance électrique générée par le passage des particules biologiques à travers l'orifice d'injection (16), le dispositif de mesure de variation d'impédance électrique comprenant une première et une deuxième électrodes (91, 92) disposées respectivement de part et d'autre de l'orifice d'injection (16), les première et deuxième électrodes (91, 92) étant destinées à être en contact électrique avec le flux de particules biologiques.

13. Système de cytométrie en flux selon l'une des revendications 1 à 12, dans lequel le dispositif d'évacuation (13) comporte :
- un conduit d'évacuation (31) relié fluidiquement à la chambre de mesure (11) et destiné à évacuer le flux de particules biologiques injecté dans la chambre de mesure, et
- le troisième conduit d'alimentation (33).

14. Ensemble de cytométrie en flux (2) comprenant au moins un système de cytométrie en flux (4) selon l'une quelconque des revendications 1 à 13.

15. Dispositif d'analyse (97) pour diagnostic in vitro, comprenant un ensemble de cytométrie en flux (2) selon la revendication 14.

16. Ensemble comprenant un système de cytométrie en flux selon l'une quelconque des revendications 1 à 13 et un banc de réglage (3) sur lequel est destiné à être monté ledit système de cytométrie en flux, dans lequel le banc de réglage (3) comporte au moins un premier dispositif de réglage en translation (64) agencé pour régler en translation la position du dispositif d'émission (42) par rapport au support de référence (6).

17. Ensemble selon la revendication 16, dans lequel le banc de réglage (3) comporte au moins un deuxième dispositif de réglage en translation (64') agencé pour régler en translation la position de l'au moins un dispositif de collecte (43a-43c) par rapport au support de référence (6).

## Patentansprüche

1. Durchflusszytometriesystem (4) umfassend:
- eine Messzelle (9), die mindestens teilweise eine Messkammer (11) eingegrenzt,
- eine Injektionsvorrichtung (12), die eingerichtet ist, um einen zu analysierenden Fluss biologischer Partikel in die Messkammer (11) zu injizieren, wobei die Injektionsvorrichtung (12) umfasst:
- eine Injektionsdüse (14), die eine innere Kammer (15) eingegrenzt, und eine mit der Messkammer (11) fluidisch verbundene Injektionsöffnung (16) umfasst,
- eine erste Versorgungsleitung (17), die in die innere Kammer (15) mündet und bestimmt ist, die innere Kammer mit einer flüssigen Probe zu versorgen, die die zu analysierenden biologischen Partikel enthält, und
- eine zweite Versorgungsleitung (19), die in die innere Kammer (15) mündet und bestimmt ist, die innere Kammer mit einer ersten Hüllflüssigkeit zu versorgen, wobei die Injektionsdüse (14) und die zweite Versorgungsleitung (19) so konfiguriert sind, dass die erste Hüllflüssigkeit, die in die innere Kammer eingeführt wird, geeignet ist, die in die innere Kammer eingeführte flüssige Probe hydrodynamisch zu umhüllen,
- eine Evakuierungsvorrichtung (13), die eingerichtet ist, um den in die Messkammer (11) injizierten Fluss von zu analysierenden biologischen Partikeln nach außerhalb des Zytometriesystems zu evakuieren,
- eine dritte Versorgungsleitung (33), die fluidisch mit der Messkammer (11) verbunden ist und bestimmt ist, die Messkammer mit einer zweiten Hüllflüssigkeit zu versorgen, wobei die Messkammer (11) und die dritte Versorgungsleitung (33) so konfiguriert sind, dass die zweite Hüllflüssigkeit, die in die Messkammer (11) eingeführt wird, geeignet ist, den Fluss biologischer Partikel in der Messkammer (11) hydrodynamisch zu umhüllen,
- eine Messanordnung, die eingerichtet ist, um mindestens eine optische Eigenschaft der zu analysierenden biologischen Partikel zu messen, wobei die Messanordnung beinhaltet:
- mindestens eine Emissionsvorrichtung (42), die eingerichtet ist, um ein Lichtbündel in Richtung der Messkammer (11) zu emittieren, und geeignet ist, den Fluss biologischer Partikel zu kreuzen, wobei mindestens eine Emissionsvorrichtung (42) eine Lichtquelle (44) umfasst, die eingerichtet ist, um das Lichtbündel zu erzeugen,
- mindestens eine Sammelvorrichtung (43a-43c), die eingerichtet ist, um die Lichtstrahlen, die aus der Messkammer (11) hervorgegangen sind, zu sammeln,
**dadurch gekennzeichnet, dass** das Durchflusszytometriesystem ferner ein einteiliges Referenzauflager (6) umfasst, auf dem die Injektionsvorrichtung (12), die Evakuierungsvorrichtung (13), die mindestens eine Emissionsvorrichtung (42) und die mindestens eine Sammelvorrichtung (43a-43c) montiert sind, und dass das Referenzauflager (6) einen Aufnahmesitz (7) eingegrenzt, in dem die Messzelle (9) untergebracht ist, wobei die Messzelle (9) fluidisch vom Aufnahmesitz (7) isoliert ist, und dass die Injektions- und Evakuierungsvorrichtungen (12, 13) jeweils an zwei dem Referenzauflager (6) gegenüberliegenden Außenseiten befestigt sind, wobei die Evakuierungsvorrichtung (13) am Referenzauflager (6) gegenüber der Injektionsvorrichtung (12) relativ zur Messzelle (9) montiert ist.

2. Durchflusszytometriesystem nach Anspruch 1, wobei die mindestens eine Emissionsvorrichtung (42) eine Fokussiervorrichtung (45) beinhaltet, die eingerichtet ist, um das Lichtbündel in der Messkammer (11) zu fokussieren und auf den Fluss biologischer Partikel (F).

3. Durchflusszytometriesystem nach Anspruch 2, wobei die Fokussiervorrichtung (45) umfasst:
- ein erstes Montageteil (46), das mit einem optischen Fokussierelement (49) ausgestattet ist, das auf dem optischen Pfad des Lichtbündels angeordnet ist,
- ein zweites Montageteil (47), auf dem die Lichtquelle (44) montiert ist, wobei das erste und das zweite Montageteil (46, 47) der Fokussiervorrichtung (45) relativ zueinander in einer ersten Verschiebungsrichtung (D1) im Wesentlichen parallel zum optischen Pfad des Lichtbündels verschiebbar sind, und
- ein erstes Anpassungselement (57), das eingerichtet ist, um die relative Position des ersten und zweiten Montageteils (46, 47) der Fokussiervorrichtung (45) entlang der ersten Verschiebungsrichtung (D1) anzupassen.

4. Durchflusszytometriesystem nach Anspruch 3, wobei die Fokussiervorrichtung (45) mindestens ein Immobilisierungselement (54) umfasst, das eingerichtet ist, um das erste Montageteil (46) relativ zum Referenzauflager (6) zu immobilisieren, wobei das zweite Montageteil (46) der Fokussiervorrichtung (45) relativ zum ersten Montageteil (46) der Fokussiervorrichtung (45) beweglich montiert ist.

5. Durchflusszytometriesystem nach einem der Ansprüche 1 bis 4, eine Ausrichtungsanpassungsvorrichtung (61) beinhaltend, die eingerichtet ist, um die Ausrichtung des Lichtbündels anzupassen, das von der mindestens einen Emissionsvorrichtung (42) emittiert wurde.

6. Durchflusszytometriesystem nach Anspruch 5, wobei die Ausrichtungsanpassungsvorrichtung (61) beinhaltet:
- ein Anpassungskissen (62), das zwischen dem Referenzauflager (6) und der mindestens einen Emissionsvorrichtung (42) angeordnet ist, wobei das Anpassungskissen (62) mindestens teilweise elastisch verformbar ist, und
- eine Verformungsanordnung, die eingerichtet ist, um das Anpassungskissen (62) zu verformen, um die Ausrichtung des Lichtbündels anzupassen, das von der mindestens einen Emissionsvorrichtung (42) emittiert wurde.

7. Durchflusszytometriesystem nach Anspruch 6 in Kombination mit Anspruch 3, wobei der erste Montageabschnitt (46) der Fokussiervorrichtung (45) einen Stützabschnitt (52) beinhaltet, der eingerichtet ist, um sich gegen das Anpassungskissen (62) abzustützen.

8. Durchflusszytometriesystem nach Anspruch 7 in Kombination mit Anspruch 4, wobei die Verformungsanordnung durch mindestens ein Immobilisierungselement und den Stützabschnitt (52) des ersten Montageabschnitts (46) gebildet wird.

9. Durchflusszytometriesystem nach einem der Ansprüche 1 bis 8, wobei die mindestens eine Sammelvorrichtung (43a-c) beinhaltet:
- ein erstes Montageteil (75), das ein erstes optisches Sammelelement (78) umfasst,
- ein zweites Montageteil (76), das mindestens ein zweites optisches Sammelelement (86) umfasst, wobei das erste und das zweite Montageteil (75, 76) der mindestens einen Sammelvorrichtung in einer zweiten Verschiebungsrichtung relativ zueinander verschiebbar sind, und
- ein zweites Anpassungselement (87), das eingerichtet ist, um die relative Position des ersten und zweiten Montageteils (75, 76) der mindestens einen Sammelvorrichtung (43a-43c) entlang der zweiten Verschiebungsrichtung anzupassen.

10. Durchflusszytometriesystem nach Anspruch 9, wobei die mindestens eine Sammelvorrichtung (43a-43c) mindestens ein Immobilisierungselement (83) umfasst, das eingerichtet ist, um das erste Montageteil (75) der Sammelvorrichtung relativ zum Referenzauflager (6) zu immobilisieren, wobei das zweite Montageteil (76) der Sammelvorrichtung relativ zum ersten Montageteil der Sammelvorrichtung beweglich montiert ist.

11. Durchflusszytometriesystem nach einem der Ansprüche 8 bis 10, wobei mindestens ein zweites optisches Sammelelement (86) mindestens eine optische Sammelfaser umfasst.

12. Durchflusszytometriesystem nach einem der Ansprüche 1 bis 11, eine elektrische Impedanzvariationsmessvorrichtung umfassend, die eingerichtet ist, um eine elektrische Impedanzvariation zu messen, die durch den Durchgang biologischer Partikel durch die Injektionsöffnung (16) erzeugt wird, wobei die elektrische Impedanzvariationsmessvorrichtung eine erste und eine zweite Elektrode (91, 92) umfasst, die jeweils auf beiden Seiten der Injektionsöffnung (16) angeordnet sind, wobei die erste und die zweite Elektrode (91, 92) bestimmt sind, in elektrischem Kontakt mit dem Fluss biologischer Partikel zu stehen.

13. Durchflusszytometriesystem nach einem der Ansprüche 1 bis 12, wobei die Evakuierungsvorrichtung (13) beinhaltet:
- eine Evakuierungsleitung (31), die fluidisch mit der Messkammer (11) verbunden ist, und bestimmt ist, um den in die Messkammer injizierten Fluss biologischer Partikel nach außerhalb zu evakuieren, und
- die dritte Versorgungsleitung (33).

14. Durchflusszytometrieanordnung (2), mindestens ein Durchflusszytometriesystem (4) nach einem der Ansprüche 1 bis 13 umfassend.

15. Analysevorrichtung (97) für In-vitro-Diagnostik, eine Durchflusszytometrieanordnung (2) nach Anspruch 14 umfassend.

16. Anordnung, ein Durchflusszytometriesystem nach einem der Ansprüche 1 bis 13 umfassend, und eine Anpassungsbank (3), die bestimmt ist, um das Durchflusszytometriesystem darauf zu montieren, wobei die Anpassungsbank (3) mindestens eine erste Vorrichtung zur translatorischen Anpassung (64) beinhaltet, die eingerichtet ist, um die Position der Emissionsvorrichtung (42) relativ zum Referenzauflager (6) translatorisch anzupassen.

17. Anordnung nach Anspruch 16, wobei die Anpassungsbank (3) mindestens eine zweite Vorrichtung zur translatorischen Anpassung (64') beinhaltet, die eingerichtet ist, um die Position der mindestens einen Sammelvorrichtung (43a-43c) relativ zum Referenzauflager (6) translatorisch anzupassen.

## Claims

1. A flow cytometry system (4) comprising:
- a measuring cell (9) delimiting at least partially a measuring chamber (11),
- an injection device (12) arranged to inject a flow of biological particles to be analyzed in the measuring chamber (11), the injection device (12) comprising:
- an injection nozzle (14) delimiting an internal chamber (15) and comprising an injection orifice (16) fluidly connected to the measuring chamber (11),
- a first feeding conduit (17) opening into the internal chamber (15) and intended to feed the internal chamber with a liquid sample containing the biological particles to be analyzed, and
- a second feeding conduit (19) opening into the internal chamber (15) and intended to feed the internal chamber with a first sheathing fluid, the injection nozzle (14) and the second feeding conduit (19) being configured so that the first sheathing fluid introduced in the internal chamber is capable of hydro-dynamically sheathing the liquid sample introduced in the internal chamber,
- an evacuation device (13) arranged to evacuate outside of the cytometry system the flow of biological particles injected in the measuring chamber (11),
- a third feeding conduit (33) fluidly connected to the measuring chamber (11) and intended to feed the measuring chamber with a second sheathing fluid, the measuring chamber (11) and the third feeding conduit (33) being configured so that the second sheathing fluid introduced in the measuring chamber (11) is capable of hydro-dynamically sheathing the flow of biological particles in the measuring chamber (11),
- a measuring set arranged to measure at least one optical property of the biological particles to be analyzed, the measuring set including:
- at least one emission device (42) arranged to emit a light beam toward the measuring chamber (11) and capable of crossing the flow of biological particles, the at least one emission device (42) comprising a light source (44) arranged to generate the light beam,
- at least one collecting device (43a-43c) arranged to collect light rays coming from the measuring chamber (11),
**characterized in that** the flow cytometry system further comprises a reference support (6) which is in one-piece and on which the injection device (12), the evacuation device (13), the at least one emission device (42) and the at least one collecting device (43a-43c) are mounted, **in that** the reference support (6) delimits a receiving housing (7) in which the measuring cell (9) is housed, the measuring cell (9) being fluidly isolated from the receiving housing (7), and **in that** the injection and evacuation devices (12, 13) are respectively fastened on two opposite external faces of the reference support (6), the evacuation device (13) being mounted on the reference support (6) opposite to the injection device (12) with respect to the measuring cell (9).

2. The flow cytometry system according to claim 1, wherein the at least one emission device (42) includes a focusing device (45) arranged to focus the light beam in the measuring chamber (11) and on the flow of biological particles (F).

3. The flow cytometry system according to claim 2, wherein the focusing device (45) comprises:
- a first mounting portion (46) equipped with an optical focusing element (49) disposed on the optical path of the light beam,
- a second mounting portion (47) on which the light source (44) is mounted, the first and second mounting portions (46, 47) of the focusing device (45) being displaceable relative to each other according to a first direction of displacement (D1) substantially parallel to the optical path of the light beam, and
- a first adjusting element (57) arranged to adjust the relative position of the first and second mounting portions (46, 47) of the focusing device (45) along the first direction of displacement (D1).

4. The flow cytometry system according to claim 3, wherein the focusing device (45) comprises at least one immobilizing element (54) arranged to immobilize the first mounting portion (46) with respect to the reference support (6), the second mounting portion (46) of the focusing device (45) being movably mounted relative to the first mounting portion (46) of the focusing device (45).

5. The flow cytometry system according to any one of claims 1 to 4, which includes an orientation adjusting device (61) arranged to adjust the orientation of the light beam emitted by the at least one emission device (42).

6. The flow cytometry system according to claim 5, wherein the orientation adjusting device (61) includes:
- an adjusting cushion (62) disposed between the reference support (6) and the at least one emission device (42), the adjusting cushion (62) being at least partially elastically deformable, and
- a deformation set arranged to deform the adjusting cushion (62) so as to adjust the orientation of the light beam emitted by the at least one emission device (42).

7. The flow cytometry system according to claim 6 in combination with claim 3, wherein the first mounting portion (46) of the focusing device (45) includes a bearing portion (52) arranged to bear against the adjusting cushion (62).

8. The flow cytometry system according to claim 7 in combination with claim 4, wherein the deformation set is formed by the at least one immobilizing element and the bearing portion (52) of the first mounting portion (46).

9. The flow cytometry system according to any one of claims 1 to 8, wherein the at least one collecting device (43a-c) includes:
- a first mounting portion (75) comprising first optical collecting element (78),
- a second mounting portion (76) comprising at least one second optical collecting element (86), the first and second mounting portions (75, 76) of the at least one collecting device being displaceable relative to each other according to a second direction of displacement, and
- a second adjusting element (87) arranged to adjust the relative position of the first and second mounting portions (75, 76) of the at least one collecting device (43a-43c) along the second direction of displacement.

10. The flow cytometry system according to claim 9, wherein the at least one collecting device (43a-43c) comprises at least one immobilizing element (83) arranged to immobilize the first mounting portion (75) of said collecting device with respect to the reference support (6), the second mounting portion (76) of said collecting device being movably mounted relative to the first mounting portion of said collecting device.

11. The flow cytometry system according to any one of claims 8 to 10, wherein the at least one second optical collecting element (86) comprises at least one collecting optical fiber.

12. The flow cytometry system according to any one of claims 1 to 11, which comprises an electrical impedance variation measuring device arranged to measure an electrical impedance variation generated by the passage of the biological particles through the injection orifice (16), the electrical impedance variation measuring device comprising a first and a second electrodes (91, 92) disposed respectively on either side of the injection orifice (16), the first and second electrodes (91, 92) being intended to be in electrical contact with the flow of biological particles.

13. The flow cytometry system according to any one of claims 1 to 12, wherein the evacuation device (13) includes:
- an evacuation conduit (31) fluidly connected to the measuring chamber (11) and intended to evacuate the flow of biological particles injected in the measuring chamber, and
- the third feeding conduit (33).

14. A flow cytometry set (2) comprising at least one flow cytometry system (4) according to any one of claims 1 to 13.

15. An analysis device (97) for in vitro diagnosis, comprising a flow cytometry set (2) according to claim 14.

16. A set comprising a flow cytometry system according to any one of claims 1 to 13 and an adjusting bench (3) on which said flow cytometry system is intended to be mounted, wherein the adjusting bench (3) includes at least one first translation adjusting device (64) arranged to adjust in translation the position of the emission device (42) with respect to the reference support (6).

17. The set according to claim 16, wherein the adjusting bench (3) includes at least one second translation adjusting device (64') arranged to adjust in translation the position of the at least one collecting device (43a-43c) with respect to the reference support (6).
